# EUROPEAN PATENT APPLICATION

(11) **EP 4 806 456 A1**
(43) Date of publication of application: **16.09.2026**
(21) Application number: 24888026.2
(22) Date of filing: 06.11.2024
(51) Int. Cl.: A61K 38/26, A61P 3/10

(54) **GLP-1 COMPOUND FOR LOWERING SUGAR LEVELS**

(30) Priority: 06.11.2023 CN 202311464180; 19.06.2024 CN 202410794911
(71) Applicant: GAN & LEE PHARMACEUTICALS CO., LTD., Beijing 101109 (CN)
(72) Inventor: GAN, Zhongru, Beijing 101109 (CN); CHEN, Wei, Beijing 101109 (CN); ZHAO, Liyuan, Beijing 101109 (CN); ZHAO, Jing, Beijing 101109 (CN)
(74) Representative: Kraus & Lederer PartGmbB
(86) International application number: PCT/CN2024/130350
(87) International publication number: WO 2025/098415

(57) **Abstract**

A GLP-1 compound for lowering sugar levels.

## Description

### TECHNICAL FIELD

The present invention relates to the field of biopharmaceuticals and relates to a GLP-1 compound for treating type 2 diabetes.

### BACKGROUND

Diabetes is a metabolic disorder characterized by hyperglycemia. Optimal glycemic control is the therapeutic goal for patients with type 2 diabetes. Although several oral antidiabetic medications and insulin are available, a significant proportion of type 2 diabetes patients fail to achieve the recommended glycemic control targets. Glucagon-like peptide-1 (GLP-1) and its analogues and derivatives demonstrate remarkable efficacy in treating both type 1 and type 2 diabetes. Consequently, the development of a GLP-1 peptide with superior properties, along with appropriate dosing regimens and administration frequencies, is an urgent priority.

### SUMMARY

The present invention relates to a method for treating type 2 diabetes, comprising administering a compound of formula (I), or the pharmaceutically acceptable salt, amide or ester thereof, to a subject in need thereof, wherein the subject has clinical evidence of hyperglycemia; wherein the method reduces the occurrence of hyperglycemic-related adverse events (MACE).

The present invention provides a method of treating or preventing metabolic syndrome, comprising administering 0.25-100 mg of a compound of formula (I), or the pharmaceutically acceptable salt, amide or ester thereof, to a subject in need thereof, 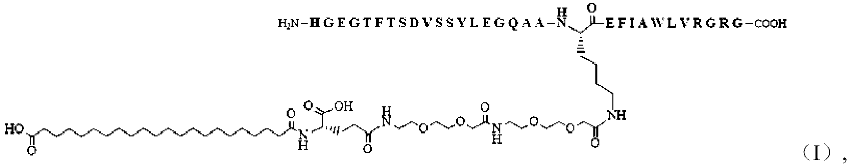
preferably, the metabolic syndrome is diabetes;
preferably, the diabetes is type 2 diabetes.

In some embodiments, wherein the method comprises administering to the subject in need thereof 0.25-100 mg, preferably 0.5-90 mg, preferably 1-80 mg, preferably 1.5-80 mg, preferably 2-80 mg, preferably 1.5-70 mg, preferably 2-70 mg, preferably 2.5-70 mg, preferably 3-70 mg, preferably 1.5-60 mg, preferably 3-60 mg, preferably 3.5-60 mg, preferably 1.5-55 mg, preferably 3-55 mg, preferably 1.5-48 mg, preferably 3-48 mg, preferably 3.5-48 mg, preferably 1.5-36 mg, preferably 3-36 mg, preferably 3.5-36 mg, preferably 1.5- 24mg, preferably 3-24mg, preferably 3.5-24mg, preferably 1.5-18mg, preferably 3-18mg, preferably 3.5-18mg, preferably 1.5-12mg, preferably 1.5-13mg, preferably 3-12mg, preferably 3.5-12mg, preferably 4-60mg, preferably 5-60mg, preferably 6-60mg, preferably 7-60mg, preferably 8-60mg, preferably 9-60mg, preferably 10-60mg, preferably 11-60mg, preferably 12-60mg, preferably 12-48mg, preferably 12-36mg, preferably 12-18mg, preferably 12-24mg of the compound of formula (I), or the pharmaceutically acceptable salt, amide or ester thereof.

In some embodiments, wherein, the method comprises administering the compound of formula (I), or the pharmaceutically acceptable salt, amide, or ester thereof, to the subject in need thereof at a dosage of 0.25-100 mg , preferably 0.5-90 mg, preferably 1.5-80 mg, preferably 1-80 mg, preferably 1.5-70 mg, preferably 2-80 mg, preferably 2-70 mg, preferably 2.5-70 mg, preferably 3-70 mg, preferably 1.5-60 mg, preferably 3-60 mg, preferably 3.5-60 mg, preferably 1.5-55 mg, preferably 3-55 mg, preferably 1.5-48 mg, preferably 3.5-48 mg, preferably 3-48 mg, preferably 1.5-36 mg, preferably 3-36 mg, preferably 3.5-36 mg, preferably 1.5-24 mg, preferably 3-24 mg, preferably 3.5-24 mg, preferably 1.5-18 mg, preferably 3-18 mg, preferably 3.5-18 mg, preferably 1.5-12 mg, preferably 1.5-13 mg, preferably 3-12 mg, preferably 3.5-12 mg, preferably 4-60 mg, preferably 5-60 mg, preferably 7-60 mg, preferably 8-60 mg, preferably 9-60 mg, preferably 10-60 mg, preferably 11-60 mg, preferably 12-60 mg, preferably 12-48 mg, preferably 12-36 mg, preferably 12-18 mg, preferably 12-24 mg per week or every two weeks.

In some embodiments, wherein, the method comprises administering to the subject in need thereof the compound of formula (I), or the pharmaceutically acceptable salt, amide or ester thereof, at the following doses per week or every two weeks: 0.25 mg, 0.5 mg, 1 mg, 1.5 mg, 2 mg, 2.5 mg, 3 mg, 3.5 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25mg, 26mg, 27mg, 28mg, 29mg, 30mg, 31mg, 32mg, 33mg, 34mg, 35mg, 36mg, 37mg, 38mg, 39mg, 40mg, 41mg, 42mg, 43mg, 44mg, 45mg, 46mg, 47mg, 48mg, 49mg, 50mg, 51mg, 52mg, 53mg, 54mg, 55mg, 56mg, 57mg, 58mg, 59mg, 60mg, 61mg, 62mg, 63mg, 64mg, 65mg, 66mg, 67mg, 68mg, 69mg, or 70mg.

In some embodiments, wherein, the method comprises administering the compound of formula (I), or the pharmaceutically acceptable salt, amide or ester thereof, to the subject in need thereof once weekly or less frequently, preferably once every two weeks or less frequently.

In some embodiments, wherein, the method comprises administering the compound of formula (I), or the pharmaceutically acceptable salt, amide, or ester thereof to the subject in need thereof at the following doses once weekly or once every two weeks: 0.25 mg, 0.5 mg, 1 mg, 1.5 mg, 2 mg, 2.5 mg, 3 mg, 3.5 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, or 70mg.

In some embodiments, wherein, the method comprises first administering an escalation dose to the subject in need thereof at a frequency of once weekly or once every two weeks during an escalation dose period, and then administering a maintenance dose at a frequency of once weekly or once every two weeks during a maintenance dose period, wherein
the escalation dose period is at least two weeks, preferably about 2-60 weeks, preferably about 2-55 weeks, preferably about 2-50 weeks, preferably about 2-40 weeks, preferably about 2-31 weeks, preferably about 2-30 weeks, preferably about 2-20 weeks, preferably the escalation dose period is about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, about 52 weeks, about 53 weeks, about 54 weeks, about 55 weeks, about 56 weeks, about 57 weeks, about 58 weeks, about 59 weeks, or about 60 weeks;
the maintenance dose period is at least two weeks, preferably about 2-60 weeks, preferably about 2-55 weeks, preferably about 2-50 weeks, preferably about 2-40 weeks, preferably about 2-35 weeks, preferably about 2-30 weeks, preferably about 2-20 weeks, preferably the escalation dose period is about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, about 52 weeks, about 53 weeks, about 54 weeks, about 55 weeks, about 56 weeks, about 57 weeks, about 58 weeks, about 59 weeks, or about 60 weeks;
the dose at each administration during the dose escalation is independently 0.25mg-70mg, preferably 1.5mg-60mg, preferably 1.5mg-48mg, preferably 1.5mg-36mg, preferably 1.5mg-24mg, preferably 1.5mg-24mg, preferably1.5mg-18mg, preferably 1.5mg-13mg, preferably 1.5mg-12mg of the compound of formula (I), or the pharmaceutically acceptable salt, amide or ester thereof; preferably, the dose at each administration during the dose escalation is independently 0.25mg, 0.5mg, 1mg, 1.5mg, 2mg, 2.5mg, 3mg, 3.5mg, 4mg, 5mg, 6mg, 7mg, 8mg, 9mg, 10mg, 11mg, 12mg, 13mg, 14mg, 15mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, or 70 mg of the compound of formula (I), or the pharmaceutically acceptable salt, amide or ester thereof;
the maintenance dose is 3-70 mg, preferably 6 mg-60 mg, preferably 9 mg-48 mg, preferably 12 mg-48 mg, preferably 12 mg-36 mg, preferably 12 mg-24 mg, preferably 12 mg to 18 mg, preferably 13 mg to 24 mg, preferably 13 mg to 36 mg of the compound of formula (I), or the pharmaceutically acceptable salt, amide or ester thereof; preferably, the maintenance dose is 3 mg, 4 mg, 5 mg, 6 mg, 7 mg, 8 mg, about 9 mg, 10 mg, 11 mg, about 12 mg, 13 mg, 14 mg, 15 mg, 16 mg, 17 mg, about 18 mg, 19 mg, 20 mg, 21 mg, 22 mg, 23 mg, about 24 mg, 25 mg, 26 mg, 27 mg, 28 mg, 29 mg, 30 mg, 31 mg, 32 mg 67mg, 68mg, 69mg, or 70mg of the compound of formula (I), or the pharmaceutically acceptable salt, amide or ester thereof; and
the maintenance dose is greater than or equal to the escalation dose.

In some embodiments, wherein the escalation dose period is about 6-31 weeks, 8-31 weeks, preferably about 10-31 weeks, preferably about 10-24 weeks, preferably about 14-20 weeks, preferably about 16-20 weeks, preferably the escalation dose period is about 6 weeks, 8 weeks, 10 weeks, about 12 weeks, about 14 weeks, about 16 weeks, about 18 weeks, about 20 weeks, about 30 weeks or about 31 weeks; preferably the escalation dose period is about 10 weeks, about 14 weeks, about 16 weeks, about 18 weeks, about 20 weeks, or about 31 weeks;
the maintenance dose period is about 4 weeks or more, 5 weeks or more, about 12 weeks or more, about 14 weeks or more, about 16 weeks or more, about 18 weeks or more, about 20 weeks or more;preferably the maintenance dose period is about 4-36 weeks; preferably, the maintenance dose period is about 4 weeks, about 5 weeks, about 10 weeks, about 12 weeks, about 14 weeks, about 16 weeks, about 18 weeks, about 20 weeks, about 22 weeks, about 24 weeks, about 26 weeks, about 28 weeks, about 30 weeks, about 32 weeks, about 34 weeks or about 36 weeks; preferably, the maintenance dose period is about 4-30 weeks, preferably, the maintenance dose period is about 4 weeks, about 5 weeks, about 10 weeks, about 12 weeks, about 14 weeks, about 16 weeks, about 18 weeks, about 20 weeks, about 22 weeks, about 24 weeks, about 26 weeks, or about 30 weeks;
the dose at each administration during the dose escalation is independently about 0.5 mg, 1 mg, 1.5 mg, 2 mg, 2.5 mg, 3 mg, 3.5 mg, 4 mg, 5 mg, 6 mg, 7 mg, 9 mg, 11 mg, 12 mg, 13 mg, 15 mg, 18 mg, 24 mg, 30 mg, 36 mg, or 48 mg of the compound of formula (I), or the pharmaceutically acceptable salt, amide or ester thereof; preferably, the dose at each administration during the dose escalation is independently 1.5 mg, 3 mg, 6 mg, 9 mg, 12 mg, 18 mg, 24 mg, or about 36 mg of the compound of formula (I), or the pharmaceutically acceptable salt, amide or ester thereof; orthe dose at each administration during the dose escalation is independently 1.5 mg, 3 mg, 5 mg, 7 mg, 9 mg, 11 mg, 13 mg or 15 mg of the compound of formula (I), or the pharmaceutically acceptable salt, amide or ester thereof;
the maintenance dose is 6 mg, 12 mg, 13 mg, 18 mg, 24 mg, 30 mg, 36 mg, or 48 mg of the compound of formula (I), or the pharmaceutically acceptable salt, amide or ester thereof; and
the maintenance dose is greater than or equal to the escalation dose.

In some embodiments, wherein the method comprises first administering the escalation dose to the subject in need thereof at a frequency of once weekly or once every two weeks during an escalation dose period, and then administering a maintenance dose at a frequency of once weekly or once every two weeks during the maintenance dose period, wherein,
the escalation dose period is about 8 weeks, 10 weeks, about 14 weeks, about 16 weeks, about 18 weeks, about 20 weeks, about 30 weeks, or about 31 weeks;
the maintenance dose period is about 4 weeks or more, about 12 weeks or more, about 16 weeks or more, about 20 weeks or more; preferably, the maintenance dose period is about 4 weeks, about 5 weeks, about 10 weeks, about 12 weeks, about 14 weeks, about 16 weeks, about 18 weeks, about 20 weeks, about 22 weeks, about 24 weeks, about 26 weeks, about 28 weeks, about 30 weeks, about 32 weeks, about 34 weeks or about 36 weeks; preferably, the maintenance dose period is about 4 weeks, about 12 weeks, about 16 weeks, about 20 weeks, about 24 weeks, 26 weeks, 28 weeks, or about 30 weeks;
the dose at each administration during the dose escalation is independently 1.5 mg, 3 mg, 5 mg, 6 mg, 7 mg, 9 mg, 11 mg, 12 mg, 13 mg, 15 mg, 18 mg, 24 mg, 30 mg, or 36 mg of the compound of formula (I) or the pharmaceutically acceptable salt, amide or ester thereof;
the maintenance dose is 3 mg, 6 mg, 12 mg, 13 mg, 18 mg, 24 mg, 30 mg, 36 mg, or 48 mg of the compound of formula (I), or the pharmaceutically acceptable salt, amide or ester thereof; and
the maintenance dose is greater than or equal to the escalation dose;
preferably:
   when the maintenance dose is about 12 mg of the compound of formula (I), or the pharmaceutically acceptable salt, amide or ester thereof, the method comprises sequentially administering to the subject in need thereof 1.5 mg of the compound of formula (I), or the pharmaceutically acceptable salt, amide or ester thereof, once every two weeks for 2 weeks or more; administering 3 mg of the compound of formula (I), or the pharmaceutically acceptable salt, amide or ester thereof, once every two weeks for 2 weeks or more; administering 6 mg of the compound of formula (I), or the pharmaceutically acceptable salt, amide or ester thereof once every two weeks for 4 weeks or more; administering 9 mg of the compound of formula (I), or the pharmaceutically acceptable salt, amide or ester thereof, once every two weeks for 4 weeks or more; and administering 12 mg of the compound of formula (I), or the pharmaceutically acceptable salt, amide or ester thereof, once every two weeks for 20 weeks or more; or
   when the maintenance dose is about 18 mg of the compound of formula (I), or the pharmaceutically acceptable salt, amide or ester thereof, the method comprises sequentially administering to the subject in need thereof 1.5 mg of the compound of formula (I), or the pharmaceutically acceptable salt, amide or ester thereof, once every two weeks for 2 weeks or more; administering 3 mg of the compound of formula (I), or the pharmaceutically acceptable salt, amide or ester thereof, once every two weeks for 2 weeks or more; administering 6 mg of the compound of formula (I), or the pharmaceutically acceptable salt, amide or ester thereof, once every two weeks for 4 weeks or more; administering 9 mg of the compound of formula (I), or the pharmaceutically acceptable salt, amide or ester thereof, once every two weeks for 4 weeks or more; administering 12 mg of the compound of formula (I), or the pharmaceutically acceptable salt, amide or ester thereof, at a frequency of once every two weeks for 4 weeks or more; and administering 18 mg of the compound of formula (I), or the pharmaceutically acceptable salt, amide or ester thereof, at a frequency of once every two weeks for 16 weeks or more; or
   when the maintenance dose is about 12 mg of the compound of formula (I), or the pharmaceutically acceptable salt, amide or ester thereof, the method comprises sequentially administering to the subject in need thereof 3 mg of the compound of formula (I), or the pharmaceutically acceptable salt, amide or ester thereof, once every two weeks for 2 weeks or more; administering 6 mg of the compound of formula (I), or the pharmaceutically acceptable salt, amide or ester thereof, once every two weeks for 4 weeks or more; administering 12 mg of the compound of formula (I), or the pharmaceutically acceptable salt, amide or ester thereof, once every two weeks for 18 weeks or more; or
   when the maintenance dose is about 12 mg of the compound of formula (I), or the pharmaceutically acceptable salt, amide or ester thereof, the method comprises sequentially administering to the subject in need thereof 1.5 mg of the compound of formula (I), or the pharmaceutically acceptable salt, amide or ester thereof, once every two weeks for 4 weeks or more; administering 3 mg of the compound of formula (I), or the pharmaceutically acceptable salt, amide or ester thereof, once every two weeks for 4 weeks or more; administering 6 mg of the compound of formula (I), or the pharmaceutically acceptable salt, amide or ester thereof, once every two weeks for 4 weeks or more; administering 9 mg of the compound of formula (I), or the pharmaceutically acceptable salt, amide or ester thereof, once every two weeks for 4 weeks or more; and administering 12 mg of the compound of formula (I), or the pharmaceutically acceptable salt, amide or ester thereof, once every two weeks for 28 weeks or more or 36 weeks or more; or
   when the maintenance dose is about 18 mg of the compound of formula (I), or the pharmaceutically acceptable salt, amide or ester thereof, the method comprises sequentially administering to the subject in need thereof 1.5 mg of the compound of formula (I), or the pharmaceutically acceptable salt, amide or ester thereof, once every two weeks for 4 weeks or more; administering 3 mg of the compound of formula (I), or the pharmaceutically acceptable salt, amide or ester thereof, once every two weeks for 4 weeks or more; administering 6 mg of the compound of formula (I), or the pharmaceutically acceptable salt, amide or ester thereof, once every two weeks for 4 weeks or more; administering 9 mg of the compound of formula (I), or the pharmaceutically acceptable salt, amide or ester thereof, once every two weeks for 4 weeks or more, administering 12 mg of the compound of formula (I), or the pharmaceutically acceptable salt, amide or ester thereof, once every two weeks for 4 weeks or more; and administering 18 mg of the compound of formula (I), or the pharmaceutically acceptable salt, amide or ester thereof, once every two weeks for 24 weeks or more or 32 weeks or more ; or
   when the maintenance dose is about 15, 17, 18, 19, 21, 24 mg or 36 mg of the compound of formula (I), or the pharmaceutically acceptable salt, amide or ester thereof, the method comprises sequentially administering to the subject in need thereof 1.5 mg of the compound of formula (I), or the pharmaceutically acceptable salt, amide or ester thereof, once weekly for 1 week or more; administering 3 mg of the compound of formula (I), or the pharmaceutically acceptable salt, amide or ester thereof, once weekly for 2 weeks or more; administering 5 mg of the compound of formula (I), or the pharmaceutically acceptable salt, amide or ester thereof, once weekly for 2 weeks or more; administering 7 mg of the compound of formula (I), or the pharmaceutically acceptable salt, amide or ester thereof, once weekly for 4 weeks or more; administering 9 mg of the compound of formula (I), or the pharmaceutically acceptable salt, amide or ester thereof, once weekly for 4 weeks or more; administering 11 mg of the compound of formula (I), or the pharmaceutically acceptable salt, amide or ester thereof, once weekly for 4 weeks or more; or administering 13 mg of the compound of formula (I), or the pharmaceutically acceptable salt, amide or ester thereof, once weekly for 4 weeks or more; or
   when the maintenance dose is about 24 mg of the compound of formula (I), or the pharmaceutically acceptable salt, amide or ester thereof, the method comprises sequentially administering to the subject in need thereof 3 mg of the compound of formula (I), or the pharmaceutically acceptable salt, amide or ester thereof, once every two weeks for 2 weeks or more; administering 6 mg of the compound of formula (I), or the pharmaceutically acceptable salt, amide or ester thereof, once every two weeks for 4 weeks or more; administering 12 mg of the compound of formula (I), or the pharmaceutically acceptable salt, amide or ester thereof, once every two weeks for 4 weeks or more; administering 24 mg of the compound of formula (I), or the pharmaceutically acceptable salt, amide or ester thereof, once every two weeks for 14 weeks or more; or
   when the maintenance dose is about 48 mg of the compound of formula (I), or the pharmaceutically acceptable salt, amide or ester thereof, the method comprises sequentially administering to the subject in need thereof 3 mg of the compound of formula (I), or the pharmaceutically acceptable salt, amide or ester thereof, once every two weeks for 2 weeks or more; administering 6 mg of the compound of formula (I), or the pharmaceutically acceptable salt, amide or ester thereof, once every two weeks for 4 weeks or more; administering 12 mg of the compound of formula (I), or the pharmaceutically acceptable salt, amide or ester thereof, once every two weeks for 4 weeks or more; administering 24 mg of the compound of formula (I), or the pharmaceutically acceptable salt, amide or ester thereof, once every two weeks for 4 weeks or more; administering 36 mg of the compound of formula (I), or the pharmaceutically acceptable salt, amide or ester thereof, once every two weeks for 4 weeks or more; administering 48 mg of the compound of formula (I), or the pharmaceutically acceptable salt, amide or ester thereof, once every two weeks for 12 weeks or more; or when the maintenance dose is about 36 mg of the compound of formula (I), or the pharmaceutically acceptable salt, amide or ester thereof, the method comprises sequentially administering to the subject in need thereof 3 mg of the compound of formula (I), or the pharmaceutically acceptable salt, amide or ester thereof, once every two weeks for 2 weeks or more; administering 6 mg of the compound of formula (I), or the pharmaceutically acceptable salt, amide or ester thereof, once every two weeks for 4 weeks or more; administering 12 mg of the compound of formula (I), or the pharmaceutically acceptable salt, amide or ester thereof, once every two weeks for 4 weeks or more; administering 24 mg of the compound of formula (I), or the pharmaceutically acceptable salt, amide or ester thereof, once every two weeks for 4 weeks or more; and administering 36 mg of the compound of formula (I), or the pharmaceutically acceptable salt, amide or ester thereof, once every two weeks for 10 weeks or more.

In some embodiments, wherein the method comprises administering to the subject in need thereof a first dose of 0.25-5 mg of the compound of formula (I), or the pharmaceutically acceptable salt, amide or ester thereof, once weekly or once every two weeks;
after at least 1 week of administration of the first dose, administering a second dose of 3-12 mg of the compound of formula (I), or the pharmaceutically acceptable salt, amide or ester thereof, is administered once weekly or once every two weeks; preferably, after 1, 2, 3 or 4 weeks of administration of the first dose, administering the second dose of 3-12 mg of the compound of formula (I), or the pharmaceutically acceptable salt, amide or ester thereof, once weekly or once every two weeks;
after the second dose is administered for 2, 3, 4, 5, 6, 7, or 8 weeks, administering a third dose of 6-24 mg of the compound of formula (I), or the pharmaceutically acceptable salt, amide or ester thereof, once weekly or once every two weeks.

In some embodiments, wherein, after administering the third dose for 2, 3, 4, 5, 6, 7, 8, 9, 10, 11, 12, 13, 14, 15, 16, 17, 18, 19, or 20 weeks, administering a fourth dose of 9-48 mg of the compound of formula (I) or the pharmaceutically acceptable salt, amide or ester thereof once weekly or once every two weeks.

In some embodiments, wherein, after administering the fourth dose for 2, 3, 4, 5, 6, 7, 8, 9, 10, 11, 12, 13, 14, 15, 16, 17, 18, 19, or 20 weeks, administering a fifth dose of 12-60 mg of the compound of formula (I) or the pharmaceutically acceptable salt, amide or ester thereof once weekly or once every two weeks.

In some embodiments, wherein, after administering the fifth dose for 2, 3, 4, 5, 6, 7, 8, 9, 10, 11, 12, 13, 14, 15, 16, 17, 18, 19, or 20 weeks, administering a sixth dose of 18-72 mg of the compound of formula (I) or the pharmaceutically acceptable salt, amide or ester thereof once weekly or once every two weeks.

In some embodiments, wherein after administering the sixth dose for 2, 3, 4, 5, 6, 7, 8, 9, 10, 11, 12, 13, 14, 15, 16, 17, 18, 19, or 20 weeks, administering a seventh dose of 24-75 mg of the compound of formula (I) or the pharmaceutically acceptable salt, amide or ester thereof once weekly or once every two weeks.

In some embodiments, wherein, the first dose is 0.5-5 mg, preferably 1-4.5 mg, preferably 1.5-4 mg, preferably 1.5-3.5 mg, preferably 1.5-3.0 mg, preferably 2-3 mg, preferably 1.5 mg or 3 mg;

In some embodiments, the second dose is 3-12 mg, preferably 3.5-7.5 mg, preferably 4-7 mg, preferably 4.5-6.5 mg, preferably 5-6 mg, preferably 3 mg or 6 mg;

In some embodiments, the third dose is 6-20 mg, preferably 6-18 mg, preferably 6-12 mg, preferably 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, preferably 6 mg or 12 mg;

In some embodiments, the fourth dose is 9-36 mg, preferably 12-36 mg, preferably 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, or 36 mg, preferably 9 mg or 24 mg;

In some embodiments, the fifth dose is 12-48 mg, preferably 18-48 mg, preferably 18-36 mg, preferably 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg or 48 mg, preferably 12 mg or 36 mg;

In some embodiments, the sixth dose is 18-60 mg, preferably 24-60 mg, preferably 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, or 60 mg, preferably 18 mg or 48 mg;

In some embodiments, the seventh dose is 24-70 mg, preferably 24-60 mg, preferably 24-48 mg, preferably 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, or 70 mg, preferably 24 mg or 48 mg.

In some embodiments, wherein, the method comprises administering a first dose of 3 mg of the compound of formula (I), or the pharmaceutically acceptable salt, amide or ester thereof, to the subject in need thereof at a frequency of once weekly or once every two weeks;
after administering the first dose for 2 weeks, administering a second dose of 6 mg of the compound of formula (I), or the pharmaceutically acceptable salt, amide or ester thereof, once weekly or once every two weeks; and
after administering the second dose for 4 weeks, administering a third dose of 12 mg of the compound of formula (I), or the pharmaceutically acceptable salt, amide or ester thereof, once weekly or once every two weeks; preferably, administering the third dose of 12 mg of the compound of formula (I), or the pharmaceutically acceptable salt, amide or ester thereof, once weekly or once every two weeks for 2 weeks or more, preferably 4 weeks or more, preferably 8 weeks or more, preferably 12 weeks or more, preferably 16 weeks or more, preferably 18 weeks or more, preferably 20 weeks or more; preferably, administering the third dose of 12 mg of the compound of formula (I), or the pharmaceutically acceptable salt, amide or ester thereof, once weekly or once every two weeks for 18 weeks.

In some embodiments, wherein, the method comprises administering a first dose of 3 mg of the compound of formula (I), or the pharmaceutically acceptable salt, amide or ester thereof, to the subject in need thereof once weekly or once every two weeks;
after administering the first dose for 2 weeks, administering a second dose of 6 mg of the compound of formula (I), or the pharmaceutically acceptable salt, amide or ester thereof, once weekly or once every two weeks;
after administering the second dose for 4 weeks, administering a third dose of 12 mg of the compound of formula (I), or the pharmaceutically acceptable salt, amide or ester thereof, once weekly or once every two weeks; and
after administering the third dose for 4 weeks, administering a fourth dose of 24 mg of the compound of formula (I) or the pharmaceutically acceptable salt, amide or ester thereof once weekly or once every two weeks; preferably, administering the fourth dose of 24 mg of the compound of formula (I) or the pharmaceutically acceptable salt, amide or ester thereof once weekly or once every two weeks for 2 weeks or more, preferably 4 weeks or more, preferably 8 weeks or more, preferably 12 weeks or more, preferably 14 weeks or more, preferably 16 weeks or more, preferably 20 weeks or more; preferably, administering the fourth dose of 24 mg of the compound of formula (I) or the pharmaceutically acceptable salt, amide or ester thereof once weekly or once every two weeks for 14 weeks.

In some embodiments, wherein, the method comprises administering a first dose of 3 mg of the compound of formula (I), or the pharmaceutically acceptable salt, amide or ester thereof, to the subject in need thereof once weekly or once every two weeks;
after administering the first dose for 2 weeks, administering a second dose of 6 mg of the compound of formula (I), or the pharmaceutically acceptable salt, amide or ester thereof, once weekly or once every two weeks;
after administering the second dose for 4 weeks, administering a third dose of 12 mg of the compound of formula (I), or the pharmaceutically acceptable salt, amide or ester thereof, once weekly or once every two weeks; and
after the third dose is administered for 4 weeks, administering a fourth dose of 24 mg of the compound of formula (I) or the pharmaceutically acceptable salt, amide or ester thereof once weekly or once every two weeks; and
after the fourth dose is administered for 4 weeks, administering a fifth dose of 36 mg of the compound of formula (I) or the pharmaceutically acceptable salt, amide or ester thereof once weekly or once every two weeks, preferably, administering the fifth dose of 36 mg of the compound of formula (I) or the pharmaceutically acceptable salt, amide or ester thereof is administered at a frequency of once weekly or once every two weeks for 2 weeks or more, preferably 4 weeks or more, preferably 8 weeks or more, preferably 10 weeks or more, preferably 12 weeks or more, preferably 14 weeks or more, preferably 16 weeks or more, preferably 20 weeks or more; preferably, administering the fifth dose of 36 mg of the compound of formula (I) or the pharmaceutically acceptable salt, amide or ester thereof at a frequency of once weekly or once every two weeks for 10 weeks.

In some embodiments, wherein, the method comprises administering a first dose of 3 mg of the compound of formula (I), or the pharmaceutically acceptable salt, amide or ester thereof, to the subject in need thereof at a frequency of once weekly or once every two weeks;
after administering the first dose for 2 weeks, administering a second dose of 6 mg of the compound of formula (I), or the pharmaceutically acceptable salt, amide or ester thereof, once weekly or once every two weeks;
after administering the second dose for 4 weeks, administering a third dose of 12 mg of the compound of formula (I), or the pharmaceutically acceptable salt, amide or ester thereof, once weekly or once every two weeks;
after administering the third dose for 4 weeks, administering a fourth dose of 24 mg of the compound of formula (I) or the pharmaceutically acceptable salt, amide or ester thereof at a frequency of once weekly or once every two weeks;
after the fourth dose is administered for 4 weeks, administering a fifth dose of 36 mg of the compound of formula (I) or the pharmaceutically acceptable salt, amide or ester thereof once weekly or once every two weeks; and
after the fifth dose is administered for 4 weeks, administering a sixth dose of 48 mg of the compound of formula (I) or the pharmaceutically acceptable salt, amide or ester thereof once weekly or once every two weeks; preferably, administering the sixth dose of 48 mg of the compound of formula (I) or the pharmaceutically acceptable salt, amide or ester thereof once weekly or once every two weeks for 2 weeks or more, preferably 4 weeks or more, preferably 8 weeks or more, preferably 12 weeks or more, preferably 16 weeks or more, preferably 20 weeks or more; preferably, administering the sixth dose of 48 mg of the compound of formula (I) or the pharmaceutically acceptable salt, amide or ester thereof once weekly or once every two weeks for 12 weeks.

In some embodiments, wherein, the method comprises administering to the subject in need thereof a first dose of 1.5 mg of the compound of formula (I), or the pharmaceutically acceptable salt, amide or ester thereof, once weekly or once every two weeks;
after administering the first dose for 4 weeks, administering a second dose of 3 mg of the compound of formula (I), or the pharmaceutically acceptable salt, amide or ester thereof, once weekly or once every two weeks;
after administering the second dose for 4 weeks, administering a third dose of 6 mg of the compound of formula (I), or the pharmaceutically acceptable salt, amide or ester thereof, once weekly or once every two weeks;
after administering the third dose for 4 weeks, administering a fourth dose of 9 mg of the compound of formula (I) or the pharmaceutically acceptable salt, amide or ester thereof once weekly or once every two weeks;
after administering the fourth dose for 4 weeks, administering a fifth dose of 12 mg of the compound of formula (I) or the pharmaceutically acceptable salt, amide or ester once weekly or once every two weeks; preferably, a fifth dose of 12 mg of the compound of formula (I) or the pharmaceutically acceptable salt, amide or ester thereof once weekly or once every two weeks for 2 weeks or more, preferably 4 weeks or more, preferably 8 weeks or more, preferably 12 weeks or more, preferably 16 weeks or more, preferably 20 weeks or more; preferably, administering the fifth dose of 12 mg of the compound of formula (I) or the pharmaceutically acceptable salt, amide or ester thereof once weekly or once every two weeks for 8 weeks or more; preferably, administering 12 mg of the compound of formula (I), or the pharmaceutically acceptable salt, amide or ester thereof once every two weeks for 28 weeks or more or 36 weeks or more.

In some embodiments, wherein, the method comprises administering to the subject in need thereof a first dose of 1.5 mg of the compound of formula (I), or the pharmaceutically acceptable salt, amide or ester thereof, once weekly or once every two weeks;
after administering the first dose for 4 weeks, administering a second dose of 3 mg of the compound of formula (I), or the pharmaceutically acceptable salt, amide or ester thereof, once weekly or once every two weeks;
after administering the second dose for 4 weeks, administering a third dose of 6 mg of the compound of formula (I), or the pharmaceutically acceptable salt, amide or ester thereof, once weekly or once every two weeks;
after administering the third dose for 4 weeks, administering a fourth dose of 9 mg of the compound of formula (I), or the pharmaceutically acceptable salt, amide or ester thereof, once weekly or once every two weeks;
after administering the fourth dose for 4 weeks, administering a fifth dose of 12 mg of the compound of formula (I) or the pharmaceutically acceptable salt, amide or ester thereof, once weekly or once every two weeks;
after the fifth dose is administered for 4 weeks, administering a sixth dose of 18 mg of the compound of formula (I) or the pharmaceutically acceptable salt, amide or ester thereof once weekly or once every two weeks, preferably administering the sixth dose of 18 mg of the compound of formula (I) or the pharmaceutically acceptable salt, amide or ester thereof once weekly or once every two weeks for 2 weeks or more, preferably 4 weeks or more, preferably 8 weeks or more, preferably 12 weeks or more, preferably 16 weeks or more, preferably 20 weeks or more; preferably administering the sixth dose of 18 mg of the compound of formula (I) or the pharmaceutically acceptable salt, amide or ester thereof once weekly or once every two weeks for 4 weeks; preferably administering 18 mg of the compound of formula (I), or the pharmaceutically acceptable salt, amide or ester thereof, once every two weeks for 24 weeks or more or 32 weeks or more.

In some embodiments, wherein, the method comprises administering to the subject in need thereof a first dose of about 1.5 mg of the compound of formula (I), or the pharmaceutically acceptable salt, amide or ester thereof, once weekly or once every two weeks;
after administering the first dose for 4 weeks, administering a second dose of about 3 mg of the compound of formula (I), or the pharmaceutically acceptable salt, amide or ester thereof, once weekly or once every two weeks;
after administering the second dose for 4 weeks, administering a third dose of about 6 mg of the compound of formula (I), or the pharmaceutically acceptable salt, amide or ester thereof, once weekly or once every two weeks;
after administering the third dose for 4 weeks, administering a fourth dose of about 9 mg of the compound of formula (I), or the pharmaceutically acceptable salt, amide or ester thereof, once weekly or once every two weeks;
after administering the fourth dose for 4 weeks, administering a fifth dose of about 12 mg of the compound of formula (I) or the pharmaceutically acceptable salt, amide or ester thereof once weekly or once every two weeks;
after administering the fifth dose for 4 weeks, administering a sixth dose of about 18 mg of the compound of formula (I) or the pharmaceutically acceptable salt, amide or ester thereof once weekly or once every two weeks;

After administering the sixth dose for 4 weeks, administering a seventh dose of about 24 mg of the compound of formula (I) or the pharmaceutically acceptable salt, amide or ester thereof once weekly or once every two weeks; preferably, administering the seventh dose of 24 mg of the compound of formula (I) or the pharmaceutically acceptable salt, amide or ester thereof once weekly or once every two weeks for 2 weeks or more, preferably 4 weeks or more, preferably 8 weeks or more, preferably 12 weeks or more, preferably 16 weeks or more, preferably 20 weeks or more; preferably, administering the seventh dose of 24 mg of the compound of formula (I) or the pharmaceutically acceptable salt, amide or ester thereof once weekly or once every two weeks for 4 weeks.

In some embodiments, wherein, the method comprises administering to the subject in need thereof a first' dose of 0.5-1.5 mg of the compound of formula (I), or the pharmaceutically acceptable salt, amide or ester thereof, once weekly or once every two weeks; and/or
after administering the first' dose for 1, 2, 3 or 4 weeks, administering a second' dose of 1-3 mg of the compound of formula (I), or the pharmaceutically acceptable salt, amide or ester thereof, once weekly or once every two weeks; and/or
after administering the second' dose for 2, 3, 4, 5, 6, 7, or 8 weeks, administering a third' dose of 2-6 mg of the compound of formula (I), or the pharmaceutically acceptable salt, amide or ester thereof, once weekly or once every two weeks; and/or
after administering the third' dose for 2, 3, 4, 5, 6, 7, or 8 weeks, administering a fourth' dose of 2.5-9 mg of the compound of formula (I), or the pharmaceutically acceptable salt, amide or ester thereof, once weekly or once every two weeks; and/or
after administering the fourth' dose for 2, 3, 4, 5, 6, 7, 8, 9, 10, 11, 12, 13, 14, 15, 16, 17, 18, 19, or 20 weeks, administering a fifth' dose of 3-12 mg of the compound of formula (I) or the pharmaceutically acceptable salt, amide or ester thereof once weekly or once every two weeks; and/or
after administering the fifth' dose for 2, 3, 4, 5, 6, 7, 8, 9, 10, 11, 12, 13, 14, 15, 16, 17, 18, 19, or 20 weeks, administering a sixth' dose of 3.5-18 mg of the compound of formula (I) or the pharmaceutically acceptable salt, amide or ester thereof once weekly or once every two weeks; and/or
after administering the sixth' dose for 2, 3, 4, 5, 6, 7, 8, 9, 10, 11, 12, 13, 14, 15, 16, 17, 18, 19, or 20 weeks, administering a seventh' dose of 4-24 mg of the compound of formula (I) or the pharmaceutically acceptable salt, amide or ester thereof once weekly or once every two weeks; and/or
after administering the seventh' dose for 2, 3, 4, 5, 6, 7, 8, 9, 10, 11, 12, 13, 14, 15, 16, 17, 18, 19, or 20 weeks, administering an eighth' dose of 6-30 mg of the compound of formula (I) or the pharmaceutically acceptable salt, amide or ester thereof once weekly or once every two weeks.

In some embodiments, wherein, the first' dose is 0.5-1.5 mg, preferably 0.5 mg, 0.6 mg, 1 mg, 1.2 mg, 1.5 mg, preferably 0.5 mg or 1.5 mg; and/or
the second' dose is 1-3 mg, preferably 1 mg, 1.5 mg, 2 mg, 2.5 mg, 3 mg, preferably 1 mg or 3 mg; and/or
the third' dose is 2-6 mg, preferably 2 mg, 3 mg, 4 mg, 5 mg, 6 mg, preferably 5 mg or 6 mg; and/or
the fourth' dose is 2.5-9 mg, preferably 2.5 mg, 3 mg, 4 mg, 5 mg, 6 mg, 7 mg, 8 mg, 9 mg, preferably 2.5 mg, 7 mg or 9 mg; and/or
the fifth' dose is 3-12 mg, preferably 7 mg, 8 mg, 9 mg, 10 mg, 11 mg, or 12 mg, preferably 3 mg, 9 mg or 12 mg; and/or
the sixth' dose is 3.5-18 mg, preferably 3.5 mg, 4 mg, 5 mg, 6 mg, 7 mg, 8 mg, 9 mg, 10 mg, 11 mg, 12 mg, 13 mg, 14 mg, 15 mg, 16 mg, 17 mg, or 18 mg, preferably 3.5 mg, 11 mg, or 18 mg; and/or
the seventh' dose is 4-24 mg, preferably 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg or 24 mg, preferably 4 mg, 18 mg or 24 mg.

In some embodiments, wherein, the method comprises administering to the subject in need thereof a first' dose of 1.5 mg of the compound of formula (I), or the pharmaceutically acceptable salt, amide or ester thereof, once weekly or once every two weeks;
after administering the first' dose for 2 weeks, administering a second' dose of 3 mg of the compound of formula (I), or the pharmaceutically acceptable salt, amide or ester thereof, once weekly or once every two weeks;
after administering the second' dose for 4 weeks, administering a third' dose of 5 mg of the compound of formula (I), or the pharmaceutically acceptable salt, amide or ester thereof, once weekly or once every two weeks;
after administering the third' dose for 4 weeks, administering a fourth' dose of 7 mg of the compound of formula (I), or the pharmaceutically acceptable salt, amide or ester thereof, once weekly or once every two weeks;
after administering the fourth' dose for 4 weeks, administering a fifth' dose of 9 mg of the compound of formula (I) or the pharmaceutically acceptable salt, amide or ester thereof once weekly or once every two weeks;
after administering the fifth' dose for 4 weeks, administering a sixth' dose of 11 mg of the compound of formula (I) or the pharmaceutically acceptable salt, amide or ester thereof once weekly or once every two weeks; and
after administering the sixth' dose for 4 weeks, administering a seventh' dose of 13 mg of the compound of formula (I) or the pharmaceutically acceptable salt, amide or ester thereof once weekly or once every two weeks.

In some embodiments, the method is characterized in that the compound of formula (I), or the pharmaceutically acceptable salt, amide or ester thereof, is administered parenterally, preferably subcutaneously.

In some embodiments, the method is characterized in that the compound of formula (I), or the pharmaceutically acceptable salt, amide or ester thereof, is administered for 12 months or more, preferably 16 months or more, preferably 18 months or more, preferably 24 months or more, preferably 28 months or more, preferably 29 months or more, preferably 30 months or more.

In some embodiments, the aforementioned method is characterized in that
the subject has a BMI of ≥18.5 kg/m², preferably has a BMI of 18.5-35 kg/m²; and/or
has an HbA1c of 7.0%-11%; and/or
has a fasting blood glucose of <15mmol/L.

In some embodiments, the aforementioned method, the compound of formula (I), or the pharmaceutically acceptable salt, amide or ester thereof, is administered in the form of a pharmaceutical composition comprising one or more pharmaceutically acceptable excipients.

In some embodiments, the aforementioned pharmaceutical composition comprises:
about 1.5 mg/ml, about 3 mg/ml, about 6 mg/ml, about 12 mg/ml, about 18 mg/ml, about 24 mg/ml, about 36 mg/ml, or about 48 mg/ml of the compound of formula (I), or the pharmaceutically acceptable salt, amide or ester thereof; preferably about 3 mg/ml, about 6 mg/ml, about 12 mg/ml, about 18 mg/ml, about 24 mg/ml, about 36 mg/ml, or about 48 mg/ml of the compound of formula (I), or the pharmaceutically acceptable salt, amide or ester thereof;
about 5 mg/ml, about 6 mg/ml, about 7 mg/ml, about 8 mg/ml, about 9 mg/ml or about 10 mg/ml of NaCl, preferably about 7 mg/ml of NaCl;
about 1 mM, 2 mM, about 3 mM, about 4 mM, about 5 mM, about 6 mM, about 7 mM, about 8 mM, 9 mM or about 10 mM of citric acid;
about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of a Na₂HPO₄ solution, preferably about 1.42 mg/ml of Na₂HPO₄; and
the pH of the pharmaceutical composition is about 7.2, about 7.3, about 7.4, about 7.5, about 7.6, about 7.7, about 7.8, about 7.9, about 8.0, about 8.1, or about 8.2, preferably about 7.3.

### Definitions

To better understand the present invention, definitions and explanations of relevant terms are provided below.

### GLP-1 analogues and GLP-1 derivatives

The term "GLP-1 analogues" or "analogues of GLP-1" as used herein refers to peptides or compounds that are variants of human glucagon-like peptide-1 (GLP-1(7-37)), wherein one or more amino acid residues of GLP-1(7-37) are substituted, and/or one or more amino acid residues are deleted, and/or one or more amino acid residues are added. Specifically, the sequence of GLP-1(7-37) is shown as SEQ ID NO: 1 in the sequence listing. Peptides having the sequence shown in SEQ ID NO: 1 may also be referred to as "native" GLP-1 or "native" GLP-1(7-37).

In the sequence listing, the first amino acid residue (histidine) of SEQ ID NO:1 is numbered 1. However, in the following text, in accordance with the established convention in the art, this histidine residue is numbered 7, and the subsequent amino acid residues are numbered accordingly, concluding with glycine as number 37. Therefore, typically, the amino acid residue numbering or position numbering for the GLP-1(7-37) sequence discussed herein refers to the sequence starting with His at position 7 and ending with Gly at position 37.

[Gly8, Arg34] GLP-1-(7-37) peptide is a GLP-1 analog wherein Gly and Arg are substituted at positions 8 and 34, respectively, relative to GLP-1(7-37) (SEQ ID NO: 1). [Arg34] GLP-1-(7-37) peptide is a GLP-1 analog having Arg at position 34 relative to GLP-1(7-37) (SEQ ID NO: 1). Specifically, the amino acid sequence of the [Gly8, Arg34] GLP-1-(7-37) peptide is shown as SEQ ID NO: 2 in the sequence listing.

In caseof GLP-1 peptides or their analogs, the term "derivative" as used herein refers to a chemically modified GLP-1 peptide or analog wherein one or more substituents are covalently bonded to said peptide. Such substituents may also be referred to as side chains.

Unless otherwise specified, references to acylation of lysine residues should be understood as occurring at the ε-amino group.

The term "peptide", when used in reference to GLP-1 analogs such as those of the present invention, denotes a compound comprising a sequence of amino acids interconnected by amide (or peptide) bonds.

In one specific embodiment, the peptide is largely or primarily composed of amino acids interconnected via amide bonds (e.g., at least 50%, 60%, 70%, 80%, or at least 90% by molar mass). In another specific embodiment, the peptide is composed of amino acids interconnected via peptide bonds.

Amino acids are molecules comprising both an amino group and a carboxyl group, optionally comprising one or more additional groups, commonly referred to as side chains.

The term "amino acid" comprises proteinogenic amino acids (encoded by the genetic code, including natural amino acids and standard amino acids), non-proteinogenic amino acids (not found in proteins, and/or not encoded in the standard genetic code), and synthetic amino acids. Non-proteinogenic amino acids are those that can be incorporated into peptides via peptide bonds but are not proteinogenic amino acids. Synthetic non-proteinogenic amino acids include those produced through chemical synthesis, namely D-isomers of amino acids encoded by the genetic code such as D-alanine and D-leucine, Aib (α-aminoisobutyric acid), Abu (α-aminobutyric acid), 3-aminomethylbenzoic acid, o-aminobenzoic acid, deamidated histidine, β-analogues of amino acids such as β-alanine, D-histidine, deamidated histidine, 2-aminohistidine, β-hydroxyhistidine, and homohistidine, etc.

Non-limiting examples of amino acids not encoded by the genetic code include γ-carboxyglutamic acid, ornithine, D-alanine, D-glutamine, and phosphoserine. Non-limiting examples of synthetic amino acids include D-isomers of amino acids, such as D-alanine and D-leucine, Aib (α-aminoisobutyric acid), β-alanine, and des-amino-histidine (desH, also known as imidopropionic acid, abbreviated as Imp).

In the following text, all amino acids not explicitly labeled as optical isomers are understood to refer to the L-isomer (unless otherwise specified).

The term "treatment" comprises therapeutic treatments, preventive treatments, and applications that reduce subjects' risk of developing diseases or other risk factors. Treatment includes, but not limited to, the complete cure of diseases, as well as the alleviation of symptoms or mitigation of underlying risks.

### BRIEF DESCRIPTION OF THE DRAWINGS

Figure 1 shows the line chart of change in body weight from baseline (±SE);
Figure 2 shows the line chart of change in BMI from baseline (±SE);
Figure 3 shows the line chart of change in waist circumference (±SE) from baseline;
Figure 4 shows the line chart of change in hip circumference from baseline (±SE);
Figure 5 shows the line chart of change in waist-to-hip ratio from baseline (±SE).

### DETAILED DESCRIPTION

The following detailed description of the implementation of the present invention will be provided with reference to specific examples. However, those skilled in the art will understand that the following examples are provided merely to illustrate the invention and should not be construed as limiting the scope of the invention. Unless otherwise specified in the examples, standard conditions or manufacturer-recommended conditions shall be followed. The reagents or instruments used for which the manufacturer is not specified are all conventional commercially available products.

### Abbreviations

AE: Adverse events
AESI: Adverse events of special interest
AUC: Area under the drug concentration-time curve
ALB: Albumin
HbA1c: Glycated hemoglobin
GLP-1: Glucagon-like peptide-1
BMI: Body Mass Index
N: Number of subjects

### Example 1

### Preparation of the title compound: N-ε²⁶-[2-(2-[2-(2-[2-(2-[4-(21-carboxyheneicosanoylamino)-4(S)-carboxybutanoylamino]ethoxy)ethoxy]acetylamino)ethoxy]ethoxy)acetyl][Gly8, Arg34]GLP-1-(7-37) peptide (Compound (I))

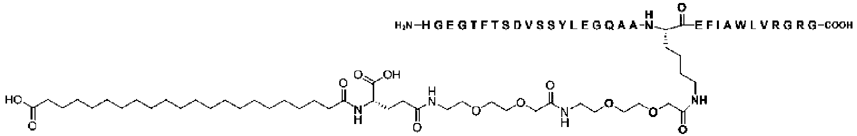

Compound I is a GLP-1 receptor agonist with CAS Registry Number: 2668298-70-0, and can be prepared with reference to, for example, the method described in WO2021136303A1.

### Example 2: Pharmaceutical Compositions of Compound (I)

Preparation of pharmaceutical composition 1 of compound (I): compound (I) at various concentrations (15 mg/3 ml or 90 mg/3 ml), 14 mg/ml propylene glycol, 1.42 mg/ml disodium hydrogen phosphate anhydrous, and pH 7.3.

Preparation of pharmaceutical composition 2 of compound (I): compound (I) at various concentrations (15 mg/3 ml or 90 mg/3 ml), 14 mg/ml propylene glycol, 1.42 mg/ml disodium hydrogen phosphate anhydrous, about 5 mM citric acid; and pH 7.3.

Preparation of pharmaceutical composition 3 of compound (I): compound (I) at various concentrations (15 mg/3 ml or 90 mg/3 ml), 8.25 mg/ml (141.17 mM) sodium chloride, 1.42 mg/ml disodium hydrogen phosphate anhydrous, about 5 mM citric acid; and pH 7.3.

### Example 3: Hypoglycemic Effect of Compound (I) Injection in Type 2 Diabetes

Evaluation of glycemic control following repeated subcutaneous injections of compound (I) injection in adult patients with type 2 diabetes mellitus.

A multicenter, randomized, controlled phase II clinical trial to evaluate the efficacy and safety of compound (I) injection in Chinese patients with type 2 diabetes mellitus was conducted. This phase II clinical trial aims to evaluate and compare glycemic control between compound (I) injection and semaglutide (Ozempic^{®}) injection following multiple subcutaneous injections in adult patients with T2DM.

### Test drug, dosage and administration, batch number:

Test Drug Name: Compound (I) Injection;
Specifications: 5 mg/mL (15 mg/3 mL), 30 mg/mL (90 mg/3 mL);
Batch Numbers: P3AI122001, P3AI423002;
Expiration Date: 2024-10 / 2025-04
Administration: Subcutaneous injection (equilibrate to room temperature prior to injection);
Manufactured and supplied by Gan & Lee Pharmaceuticals.

### Control drug name, dosage and administration, batch number:

Generic Name: Semaglutide (Ozempic ^{®});
Specifications: 1.34mg/ml (2mg/1.5ml), 1.34mg/ml (4mg/3ml);
Batch Numbers: 202303BGL1, 202302BHP2, 202211BDL1, 202210BCW2, 202211BEJ1;
Expiration Date: 36 months;
Administration: Subcutaneous injection (equilibrate to room temperature prior to injection);
Manufactured by Novo Nordisk A/S.

### Research Method:

This phase II clinical trial is a multicenter, randomized, parallel and controlled trial conducted in adult patients with type 2 diabetes mellitus (T2DM). It aims to evaluate the efficacy, safety (including immunogenicity), and pharmacokinetics of compound (I) injection in adult patients with T2DM.

Patients with type 2 diabetes who are either treatment-naive or have not received standardized therapy will be randomly assigned to receive monotherapy with the test drug. Patients with type 2 diabetes who received treatment were randomly assigned to receive the test drug on a background of metformin alone. After screening, eligible subjects were randomly assigned to four different treatment groups receiving compound (I) injection: the compound (I)-12 mg group administered once every two weeks, the compound (I)-18 mg group administered once every two weeks, the compound (I)-24 mg group administered once every two weeks, and the compound (I)-24 mg group administered once weekly, along with a control group receiving 1 mg semaglutide administered once a week (hereinafter referred to as the semaglutide group).

This study screened a total of 476 subjects, of whom 272 were successfully enrolled and 204 were excluded. Among the successfully screened subjects, 54 were assigned to the semaglutide group, 55 to the compound (I)-12 mg once every two weeks group, 54 to the compound (I)-18 mg once every two weeks group, 55 were assigned to the compound (I)-24 mg once every week group, and 54 to the compound (I)-24 mg once weekly group. Subject inclusion and exclusion criteria are detailed in Table 2.

As shown in Table 1, the compound (I) injection and semaglutide were administered to subjects according to the dosing regimen in Table 1.

### Duration of treatment:

The maximum study duration for each subject in this research was approximately 29 weeks: comprising a screening period of up to 2 weeks + a 24-week dosing treatment period (8-18 weeks for dose titration, followed by 6-16 weeks of stable-dose treatment) + a 3-week post-treatment follow up period (safety follow up conducted in the 4th week after the last dose).

**Table 1. Experimental Design: Doses of Compound (I) Injection or Semaglutide Administered at Different Experimental Stages**

| Group | Dosage frequency | Maintenance dose | Dosing schedule | | |
|---|---|---|---|---|---|
| | | | Duration | Dosing week | Dose |
| 1 | Once every two weeks | Compound (I)-12 mg | 2W | WO | 3 mg |
| | | | 4W | W2/4 | 6 mg |
| | | | 4W | W6/8 | 9 mg |
| | | | 14W | W 10/12/14/16/18/20/22 | 12 mg |
| 2 | | Compound (I)-18 mg | 2W | WO | 3 mg |
| | | | 4W | W2/4 | 6 mg |
| | | | 4W | W6/8 | 9mg |
| | | | 4W | W10/12 | 12mg |
| | | | 10W | W14/16/18/20/22 | 18mg |
| 3 | | Compound (I)-24 mg | 2W | WO | 3 mg |
| | | | 4W | W2/4 | 6 mg |
| | | | 4W | W6/8 | 9mg |
| | | | 4W | W10/12 | 12 mg |
| | | | 4W | W14/16 | 18 mg |
| | | | 6W | W18/20/22 | 24 mg |
| 4 | Once weekly | Compound (I)-24 mg | 2W | WO/1 | 3 mg |
| | | | 4W | W2/3/4/5 | 6 mg |
| | | | 4W | W6/7/8/9 | 9mg |
| | | | 4W | W10/11/12/13 | 12 mg |
| | | | 4W | W14/15/16/17 | 18 mg |
| | | | 6W | W18/19/20/21/22/23 | 24 mg |
| 5 | | Semaglutide-1.0 mg (Ozempic^{®}) | 4W | WO/1/2/3 | 0.25 mg |
| | | | 4W | W4/5/6/7 | 0.5 mg |
| | | | 16W | W8/9/10/11/12/13/14/15/16/1 7/18/19/20/21/22/23 | 1 mg |

| | | | | | |
|---|---|---|---|---|---|
| Note: W denotes week, W2/4 indicates the 2nd or 4th week. | | | | | |

**Table 2. Subject Inclusion and Exclusion Criteria (Eligible subjects must meet all inclusion criteria; subjects to be excluded meet one or more exclusion criteria)**

| | Limitations |
|---|---|
| Inclusion criteria | Subjects must meet all of the following inclusion criteria to be eligible for enrollment in this study: |
| | (1) Chinese adults aged 18 to 75 years old (including both ends, based on the date of signing the informed consent form), male or female; |
| | (2) Diagnosed with type 2 diabetes for more than three months according to the diagnostic and classification criteria for diabetes mellitus issued by the World Health Organization (WHO) in 1999, and the supplementary diagnostic criteria issued by WHO in 2011; |
| | (3) Patients with poor glycemic control who, within 3 months before screening, had lifestyle interventions and/or unregulated use of antidiabetic drugs, or who had stable use of ≤3 oral hypoglycemic drugs[including metformin, with a metformin dosage ≥1500 mg/day or at the maximum tolerated dose (<1500 mg/day but ≥1000 mg/day)] within 3 months before screening;(4) Glycated hemoglobin (HbA1c) ≥7.0% and ≤11% at screening; |
| | (5) Fasting plasma glucose <15 mmol/L at screening; |
| | (6) Body mass index (BMI) ≥ 18.5 kg/m²; |
| | (7) There is no plan within 6 months from the signing of informed consent to the last administration, and those who voluntarily take effective contraceptive measures and have no sperm donation plan. Fertile women are not breastfeeding, and the screening and baseline pregnancy tests must be negative; |
| | (8) Patients fully understand the purpose, nature, methods, and possible adverse reactions of the trail. They can communicate well with the investigator, and can understand and comply with all requirements of this study, maintain a stable diet and exercise lifestyle throughout the study period, and voluntarily sign an informed consent form to enter this study. |
| Exclusion criteria | Subjects will not be enrolled in the study if they meet any of the following criteria: |
| | (1) Known or suspected to be allergic to GLP-1 drugs or their excipients; or have of contraindications to their use; |
| | (2) Participation in any other clinical trial of a drug or device within 3 months before screening, and having received treatment; |
| | (3) Undergone surgery that may lead to weight instability within 2 months before screening; or current use of non-diabetic drugs that affect body weight; or currently following a weight loss program and not in the maintenance stage; |
| | (4) History of alcohol or drug abuse, or positive results on pre-random drug abuse screening (urine screening); |
| | (5) Continuous insulin therapy for more than 14 days in one year before screening (excluding insulin use for gestational diabetes); use of GLP-1 RA drugs within 6 months before screening; use of dipeptidyl peptidase-4 (DPP-4) inhibitors within 1 month before screening; |
| | (6) Use of growth hormone or other drugs that, in the investigator's judgment, may affect insulin levels within 3 months before screening;(7) Diabetic ketoacidosis, diabetic lactic acidosis, or hyperosmolar non-ketotic diabetic coma within 6 months before screening; |
| | (8) Proliferative diabetic retinopathy, maculopathy, severe diabetic neuropathy, intermittent claudication, or diabetic foot that is unstable or requires treatment within 6 months before screening; |
| | (9) Severe hypoglycemia (Level 3 hypoglycemia) within 6 months before screening; or 3 or more hypoglycemic events (blood glucose <3.9 mmol/L) within 1 month before screening; or recurrent symptoms associated with hypoglycemia; |
| | (10) Severe trauma, serious infection, or surgery within 1 month before screening that may affect glycemic control; |
| | (11) Calcitonin level ≥50 ng/L (pg/mL) at screening; |
| | (12) History of medullary thyroid carcinoma, multiple endocrine neoplasia (MEN) type 2A or 2B syndrome, or relevant family history; or diagnosis of any other malignancy within 5 years before screening; |
| | (13) Uncontrolled hyperthyroidism or hypothyroidism, except for patients who have been on a stable dose of thyroid hormone replacement therapy for at least 3 months before screening and have thyroid function tests (TSH/FT3/FT4) within the normal range; |
| | (14) Patients with acute or chronic hepatitis, or a history of acute or chronic pancreatitis, symptomatic gallbladder disease (e.g., multiple gallstones), pancreatic injury, or other high-risk factors that may lead to pancreatitis; |
| | (15) Uncontrolled hypertension is defined as systolic blood pressure (SBP) ≥160 mmHg and/or diastolic blood pressure (DBP) ≥100 mmHg at screening; |
| | (16) History of hospitalization for severe cardiovascular events (including but not limited to myocardial infarction, unstable angina, acute cerebrovascular disease, peripheral vascular disease) within the past 6 months before screening; or history of cerebrovascular disease with sequelae; or implanted pacemaker at screening; or patients without a pacemaker but with a 12-lead electrocardiogram (12-ECG) showing second- or third-degree atrioventricular block, long QT syndrome, or a QTc interval >450 ms; or patients with New York Heart Association functional class III or IV; or other clinically significant cardiac abnormalities determined by the investigator to make participation in this clinical study inappropriate; |
| | (17) Hematological disorders (e.g., aplastic anemia, myelodysplastic syndrome, thalassemia, sickle cell anemia, hemolytic anemia) or any condition causing hemolysis or red blood cell instability (e.g., malaria, Henoch-Schonleinpurpura); or blood donation or significant blood loss (>400 mL) or receipt of blood transfusion within 3 months before screening; |
| | (18) Clinically significant gastric emptying abnormalities (e.g., gastric outlet obstruction), severe chronic gastrointestinal diseases (e.g., active ulcers within 6 months), long-term use of drugs that directly affect gastrointestinal motility (including but not limited to domperidone, mosapride, cisapride, etc.); or those who have undergone gastrointestinal surgery within 6 months before screening, and who are deemed unsuitable for participation in this clinical study by the investigator; |
| | (19) Laboratory test results meeting any of the following criteria: Alanine aminotransferase (ALT) level ≥ 2.5 times the upper limit of normal (ULN); Aspartate aminotransferase (AST) ≥ 2.5 times ULN; fasting triglycerides (TG) >5.7 mmol/L or 500 mg/dL; serum amylase and/or lipase ≥1.5 times the upper limit of normal or clinically significant abnormalities; estimated glomerular filtration rate (eGFR) <60 mL/min/1.73m²; |
| | (20) Positive screening results for hepatitis B surface antigen (HBsAg), hepatitis C virus (HCV) antibody (except where the subject has two negative HCV RNA test results obtained at least six months apart before screening, and the third HCV RNA test obtained during screening is also negative), human immunodeficiency virus (HIV) antibody, or Treponema pallidum infection; |
| | (21) History of organ transplantation, or other acquired or congenital immune system disorders; |
| | (22) Patients with severe mental illness or those taking psychotropic drugs; |
| | (23) Other conditions that, in the investigator's judgment, make the subject unsuitable for participation in this clinical study. |

### Statistical Methods:

### 1. Analysis Set

Demographic data and protocol deviation analysis are based on the intention-to-treat (ITT) analysis set. Analysis of baseline characteristics, prior/concomitant medications and non-pharmaceutical treatments, and efficacy analysis are based on the full analysis set (FAS). Pharmaceutical exposure and adherence, safety analysis based on the safety analysis dataset (SS). PK analysis is based on the pharmacokinetic analysis dataset (PKS). Immunogenicity analysis is based on the immunogenicity analysis set (IS).

### 2. General Statistical Considerations

All statistical analyses are performed using SAS^{®} version 9.4 or a later version. Pharmacokinetic calculations are performed using Phoenix^{®} WinNonlin 8.3.5 or a later version. Descriptive statistics for quantitative data are presented using case counts (missing data), mean, standard deviation, median, quartiles (Q1 and Q3), minimum value, and maximum value. Qualitative data are described using descriptive statistics, including frequencies and percentages. Percentages are calculated using the non-missing data within each analysis set as the denominator, unless otherwise specified. Unless otherwise specified, all statistical tests are two-tailed with α=0.05, and p< 0.05 will be considered statistically significant.

### 3. Estimands Analysis

### (1) Primary Estimation Method

Based on the FAS, the primary efficacy endpoint "change in HbA1c from baseline after 24 weeks of continuous administration" was quantitatively described by treatment group. Plot line graphs showing HbA1c values at each visit and changes from baseline for each dose group, along with bar charts depicting changes in HbA1c from baseline after 24 weeks of continuous administration. The primary outcome measures were analyzed using a mixed-effects model for repeated measures (MMRM).

### (2) Sensitivity Analysis Method

Based on FAS, missing data were imputed using multiple imputation (MI), with stratified factors (treatment-naive, previously treated), baseline HbA1c levels, and baseline BMI as covariates. A covariance analysis model was employed to statistically compare differences between the experimental and control groups, incorporating the following stratified factors as covariates: treatment status (naive vs. pretreated), baseline HbA1c level, baseline BMI, relative dose intensity during the titration period, and relative dose intensity during the stable-dose treatment period.

### (3) Supplementary Analysis

The robustness of the primary estimated target outcomes was confirmed through supplementary analyses across different dimensions.

### Experimental Results and Conclusions

### 1. Subject Distribution

A total of 476 subjects were screened for this study, with 204 (42.9%) failing to meet inclusion criteria. Ultimately, 272 subjects (57.1%) were successfully randomized. All randomized subjects received at least one dose of the study medication. ITT Set: 54 subjects in the semaglutide group, 55 in the compound (I)-12 mg once every two weeks group, 54 in the compound (I)-18 mg once every two weeks group, 55 in the compound (I)-24 mg once every two weeks group, and 54 in the compound (I)-24 mg once weekly group.

FAS Set: 50 subjects in the semaglutide group, 55 in the compound (I)-12 mg once every two weeks group, 53 in the compound (I)-18 mg once every two weeks group, 54 in the compound (I)-24 mg once every two weeks group, and 52 in the compound (I)-24 mg once weekly group.

PPS Set: 40 subjects in the semaglutide group, 42 in the compound (I)-12 mg once every two weeks group, 37 in the compound (I)-24 mg once every two weeks group, 43 in the compound (I)-18 mg once every two weeks group, and 33 in the compound (I)-24 mg once weekly group.

SS Set: 54 subjects in the semaglutide group, 55 in the compound (I)-12 mg once every two weeks group, 53 in the compound (I)-18 mg once every two weeks group, 54 in the compound (I)-24 mg once every two weeks group, and 54 in the compound (I)-24 mg once weekly group.

PKS Set: 52 subjects (94.5%) in the compound (I)-12 mg once every two weeks group, 45 subjects (83.3%) in the compound (I)-18 mg once every two weeks group, 43 subjects (78.2%) in the compound (I)-24 mg once every two weeks group, and 44 subjects (81.5%) in the compound (I)-24 mg once weekly group.

IS Set: 54 subjects (98.2%) in the compound (I)-12 mg once every two weeks group, 52 subjects (96.3%) in the compound (I)-18 mg once every two weeks group, 50 subjects (90.9%) in the compound (I)-24 mg once every two weeks group, and 51 subjects (94.4%) in the compound (I)-24 mg once weekly group.

### 2. Demographics and Baseline Characteristics

The demographic and baseline characteristics of the subjects in each group were generally balanced and comparable.

In the FAS, the mean age (years) + SD for each treatment group was as follows: Semaglutide group: 50.5 + 10.45, compound (I)-12 mg once every two weeks group: 50.0 + 11.08, compound (I)-18 mg once every two weeks group: 50.3 + 9.16, compound (I)-24 mg once every two weeks group: 50.0 + 10.60, compound (I)-24 mg once weekly group: 52.4 + 11.38.

The trial enrolled two major categories of type 2 diabetes subjects: treatment-naive/non-standardized treatment and previously treated. Among the FAS, the distribution of treatment-naive subjects across groups was as follows: 22 subjects (44.0%) in the Semaglutide group; 25 subjects (45.5%) in the compound (I)-12 mg once every two weeks group; 23 subjects (43.4%) in the compound (I) 18 mg-once every two weeks group, 25 subjects (46.3%) in the compound (I)-24 mg once every two weeks group, and 24 subjects (46.2%) in the compound (I)-24 mg once weekly group.

In the FAS, the mean + SD of HbA1c (%) values across treatment groups was as follows: Semaglutide group: 8.28 + 0.933, compound (I)-12 mg once every two weeks group: 8.56 + 1.116, compound (I)-18 mg once every two weeks group: 8.31 + 1.017, compound (I)-24 mg once every two weeks group: 8.31 + 0.937, compound (I)-24 mg once weekly group: 8.28 + 1.065.

In the FAS, the mean + SD of fasting blood glucose (mmol/L) values across treatment groups was as follows: Semaglutide group: 9.997 + 2.7071; compound (I)-12 mg once every two weeks group: 9.900 + 2.2167; compound (I)-18 mg once every two weeks group: 9.628 + 1.9879. compound (I)-24 mg once every two weeks group: 9.667 ±2.2204, compound (I)-24 mg once weekly group: 10.018 +2.5190.

### 3. Efficacy Results

### HbA1c (%) Indicator

In FAS, the MMRM analysis results for the change in HbA1c (%) from baseline after 24 weeks of continuous administrationare shown in Table 3.

**Table 3 Change in HbA1c from Baseline (MMRM) FAS (N=264)**

| **Visit** | **LSMean(95%CI)** | | | | |
|---|---|---|---|---|---|
| | **Once weekly semaglutid e-1 mg group (N=50)** | **Once every two weeks compou nd (I)-** | **Once every two weeks compou nd (I)-** | **Once every two weeks compou nd (I)-** | **Once weekly compound( I)-24 mg** |
| | | **12 mg group (N=55)** | **18 mg group (N=53)** | **24 mg group (N=54)** | **group (N=52)** |
| V14W24 (%) | -1.60 (-1.85, -1.35) | -1.87 (-2.11, -1.63) | -2.28 (-2.54, -2.03) | -1.94 (-2.19, -1.69) | -2.32 (-2.57, -2.06) |
| Experiment al group - control group LSMean (95%CI) | - | -0.27 (-0.61, 0.08) | -0.68 (-1.04, -0.33) | -0.34 (-0.70, 0.02) | -0.72 (-1.08, -0.36) |
| SE | - | 0.18 | 0.18 | 0.18 | 0.18 |
| p-value | - | 0.132 | <0.001 | 0.063 | <0.001 |

| | | | | | |
|---|---|---|---|---|---|
| Note: (1) A mixed-effects model for repeated measures was used to analyze changes in HbA1c from baseline after 24 weeks of continuous administration. Missing data were not imputed in this model. Treatment groups, random stratification factors (treatment-naive, previously treated), baseline HbA1c, relative dose intensity during the titration period, relative dose intensity during the stable dose treatment period, baseline BMI, and the treatment group-visit interaction as fixed factors. | | | | | |

In the FAS, the MMRM analysis results for change from baseline in HbA1c (%) after 24 weeks of continuous administration were as follows:
The least squares mean for the Semaglutide group was -1.6 (95% CI: -1.85, -1.35). The least squares mean for the compound (I)-12 mg once every two weeks group was -1.87 (95% CI: -2.11, -1.63), experimental group-control group was -0.27 (95% CI: -0.61, -0.08, P=0.132). The least squares mean for the compound (I)-18 mg once every two weeks group was -2.28 (95% CI: -2.54, -2.03), experimental group-control group was -0.68 (95% CI: -1.04, -0.33, P < 0.001). The least squares mean for the compound (I)-24 mg once every two weeks group was -1.94 (95% CI: -2.19, -1.69), experimental group-control group was -0.34 (95% CI: -0.70, 0.02; P=0.063). The least squares mean for the compound (I)-24 mg once weekly group was -2.32 (95% CI: -2.57, -2.06), experimental group-control group was -0.72 (95%CI: -1.08, -0.36; P < 0.001). Among these, the compound (I)-18 mg once every two weeks group and the compound (I)-24 mg once weekly group demonstrated significantly superior efficacy compared to the semaglutide control group (P < 0.001).

Table 3 demonstrates that the compound (I) injection effectively reduces HbA1c levels at all doses and administration frequencies. All compound (I) dose groups demonstrated greater reductions compared to the semaglutide group. Among them, the compound (I)-18 mg once every two weeks group and the compound (I)-24 mg once weekly group showed significantly superior efficacy compared to the semaglutide group (P < 0.001).

**Table 4 HbA1c Target Achievement Rate After 24 Weeks of Continuous Administration (Exact Clopper-Pearson) - FAS (N=264)**

| **Visit** | **Once weekly semaglutide-1 mg group (N=50)** | **Once every two weeks compound (I)-12 mg group (N=55)** | **Once every two weeks compound (I)-18 mg group (N=53)** | **Once every two weeks compound (I)-24 mg group (N=54)** | **Once weekly compound( I)-24 mg group (N=52)** |
|---|---|---|---|---|---|
| V14W24 (%) (<7.0%) | 70.00 (55.39, 82.14) | 72.73 (59.04, 83.86) | 73.58 (59.67, 84.74) | 62.96 (48.74, 75.71) | 75.00 (61.05, 85.97) |
| V14W24 (%) (≤6.5%) | 62.00 (47.17, 75.35) | 58.18 (44.11, 71.35) | 67.92 (53.68, 80.08) | 59.26 (45.03, 72.43) | 69.23 (54.90, 81.28) |

| | | | | | |
|---|---|---|---|---|---|
| Note: (1) The Exact Clopper-Pearson method based on the binomial distribution was used to calculate the HbA1c target achievement rates (proportion of subjects with HbA1c <7.0% and ≤6.5%) and their 95% confidence intervals after 24 weeks of continuous administration. | | | | | |

Table 4 shows that for HbA1c target achievement rates (HbA1c < 7.0% and HbA1c ≤ 6.5%): the compound (I) 18-mg once every two weeks group, the compound (I)-18 mg once every two weeks group, and the compound (I)-24 mg once weekly group all demonstrated a trend toward superiority over the control group.

### Fasting blood glucose, insulin resistance index (HOMA-IR), pancreatic beta-cell function (HOMA-β), insulin sensitivity index (ISI)

A mixed-effects model for repeated measures (MMRM) was used to analyze changes in fasting blood glucose levels relative to baseline.

**Table 5: Changes from Baseline in Fasting Blood Glucose, Insulin Resistance Index (HOMA-IR), Pancreatic β-Cell Function (HOMA-β), and Insulin Sensitivity Index (ISI) (MMRM) FAS (N=264)**

| **Item** | **Visit** | **LSMean(95%CI)** | | | | |
|---|---|---|---|---|---|---|
| | | **Once weekly Semaglutide-1 mg group (N=50)** | **Once every two weeks compound (I)-12 mg group (N=55)** | **Once every two weeks compound (I)-18 mg group (N=53)** | **Once every two weeks compound (I)-24 mg group (N=54)** | **Once weekly compound(I)-24 mg group (N=52)** |
| **Fasting blood glucose (mmol/L)** | V14W24 | -2.23 (-2.96, - 1.49) | -2.31 (-3.00, - 1.62) | -2.74 (-3.46, - 2.02) | -2.37 (-3.09, - 1.66) | -3.87 (-4.60, - 3.15) |
| | Experimental group-control group LSMean(95%CI) | | -0.09 (-1.09, 0.92) | -0.52 (-1.56, 0.52) | -0.15 (-1.19, 0.89) | -1.65 (-2.70, - 0.60) |
| **Insulin resistance Index** | V14W24 | -0.80 (-1.94, 0.33) | -1.90 (-2.97, - 0.84) | -1.63 (-2.75, - 0.52) | -1.04 (-2.14, 0.06) | -2.58 (-3.70, - 1.46) |
| | Experimental group-control group LSMean(95%CI) | - | -1.10 (-2.66, 0.45) | -0.83 (-2.44, 0.78) | -0.24 (-1.84, 1.37) | -1.78 (-3.39, - 0.16) |
| **Pancreati c β-cell function** | V14W24 | 32.56 (1.21, 63.90) | 30.58 (1.22, 59.94) | 44.68 (13.91, 75.44) | 9.43 (-21.08, 39.94) | 53.91 (22.84, 84.98) |
| | Experimental group-control group LSMean(95%CI) | | -1.98 (-44.82, 40.86) | 12.12 (-32.41, 56.65) | -23.13 (-67.60, 21.34) | 21.35 (-23.40, 66.10) |
| **Insulin sensitivity index** | V14W24 | 0.0034 (0.0001, 0.0068) | 0.0064 (0.0033, 0.0096) | 0.0059 (0.0026, 0.0092) | 0.0035 (0.0002, 0.0067) | 0.0108 (0.0075, 0.0141) |
| | Experimental group-control group LSMean(95%CI) | - | 0.0030 (-0.0016, 0.0076) | 0.0024 (-0.0023, 0.0072) | 0.0000 (-0.0047, 0.0048) | 0.0074 (0.0027, 0.0122) |

| | | | | | | |
|---|---|---|---|---|---|---|
| Note: (1) A mixed-effects model for repeated measures was used to analyze changes in fasting blood glucose, insulin, C-peptide, glucagon, and insulin homeostasis model assessment from baseline at each visit. Missing data were not imputed in this model. Treatment groups, random stratification factors (treatment-naive, previously treated), visit (V5, V7, V9, V11, V13, V14; as categorical variables), baseline HbA1c, relative dose intensity during the titration period, relative dose intensity during the stable-dose treatment period, baseline BMI, and the treatment group-visit interaction as fixed factors. | | | | | | |

As shown in Table 5, all treatment groups demonstrated varying degrees of improvement from baseline in fasting blood glucose, insulin resistance index (HOMA-IR), pancreatic β-cell function (HOMA-β), and insulin sensitivity index (ISI). The compound (I)-18 mg once every two weeks group exhibited greater improvement trends across all parameters compared to the semaglutide group.

### Weight, BMI, waist circumference, hip circumference

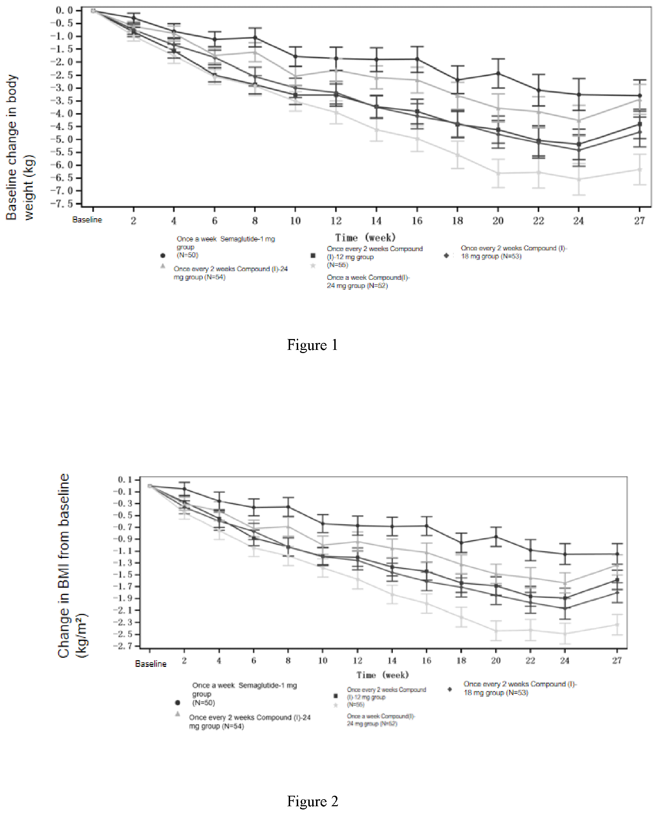

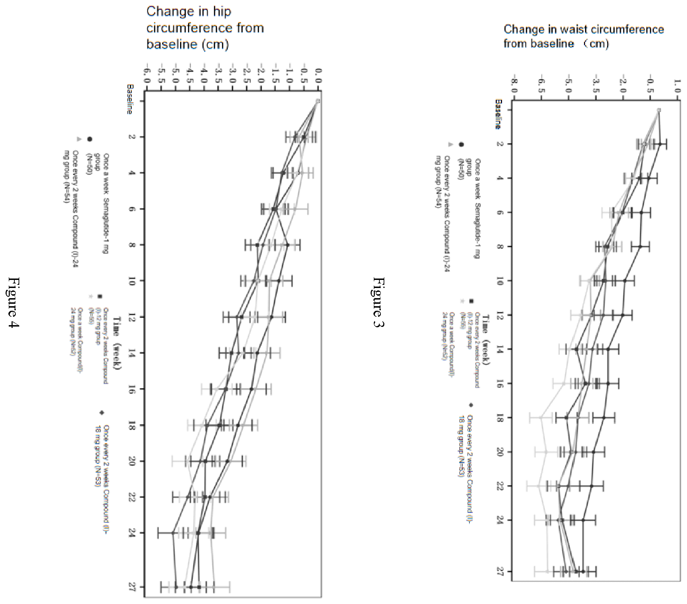

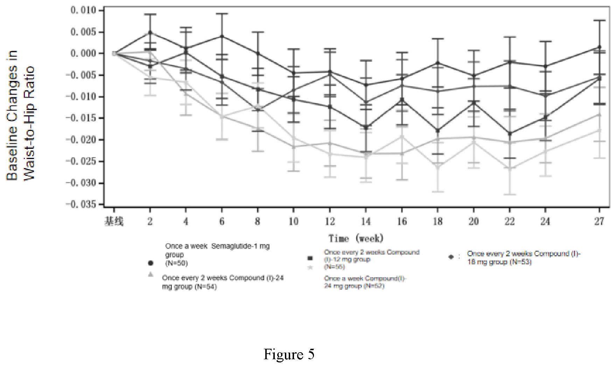

Based on the baseline changes in weight, BMI, waist circumference, hip circumference, and waist-to-hip ratio (MMRM) shown in Figures 1-5, it can be observed that all treatment groups exhibited varying degrees of reduction in weight, BMI, waist circumference, and hip circumference compared to baseline. Furthermore, the compound (I)-18 mg once every two weeks group demonstrated a greater trend of change than the semaglutide group.

### 7-point SMBG profile

A mixed-effects model was used to analyze the change in AUC/t from baseline for 7-point SMBG.

In the FAS, the change from baseline in AUC/t of 7-point SMBG (mmol/L) after 24 weeks of continuous administration was as follows:
The least squares mean for the semaglutide group was -2.78 (95% CI: -3.42, -2.15); Using the semaglutide group as the control, the least squares mean for compound (I)-12 mg once every two weeks group was -3.41 (95% CI: -4.02, -2.80), with a least squares mean difference from the control group of -0.63 (95% CI: -1.50, 0.25); The least squares mean for the compound (I)-18 mg once every two weeks group was -3.41 (95% CI: - 4.03, -2.80), with a least squares mean difference from the control group of -0.63 (95% CI: -1.51, 0.25); The least squares mean for the compound (I)-24 mg once every two weeks group was -2.51 (95% CI: -3.11, -1.90), with a least squares mean of 0.27 (95% CI: -0.60, 1.15) for the control group; The least squares mean for the compound (I)-24 mg once weekly group was -3.47 (95% CI: -4.09, -2.84), compared with a least squares mean of -0.68 (95% CI: -1.58, 0.21) for the control group, as shown in Table 6.

**Table 6 Change from Baseline in AUC/t of 7-Point SMBG (FAS, N=264)**

| **Visit** | **LSMean(95%CI)** | | | | |
|---|---|---|---|---|---|
| | **Once weekly semaglutide-1 mg group (N=50)** | **Once every two weeks GZR18-12 mg group (N=55)** | **Once every two weeks GZR18-18 mg group (N=53)** | **Once every two weeks GZR18-24 mg group (N=54)** | **Once weekly GZR18-24 mg group (N=52)** |
| V14W24 | -2.78 (-3.42, - 2.15) | -3.41 (-4.02, -2.80) | -3.41 (-4.03, - 2.80) | -2.51 (-3.11, -1.90) | -3.47 (-4.09, -2.84) |
| Experimental group - control group LSMean(95%CI) | - | -0.63 (-1.50, 0.25) | -0.63 (-1.51, 0.25) | 0.27 (-0.60, 1.15) | -0.68 (-1.58, 0.21) |

AUC/t of 7-point SMBG after 24 weeks of continuous administration (mixed-effects model): all treatment groups showed reductions from baseline. The compound (I)-12 mg once every two weeks group, the compound (I) 18-mg once every two weeks group, the compound (I)-18 mg once every two weeks group, and the compound (I) 24-mg once weekly group exhibited greater reductions than the control group.

### Adverse Reactions of Compound (I) Injection

The adverse reactions associated with compound (I) injection are consistent with those commonly observed in glucagon-like peptide-1 receptor agonists (GLP-1RAs). The most frequently reported adverse reactions, in descending order of incidence, were nausea, vomiting, decreased appetite, diarrhea, abdominal distension, weakness, and elevated lipase levels, which were similar to those of the semaglutide group.

### Medication compliance

In this study, 100% of subjects in all compound (I) treatment groups had compliance (actual dose administered / prescribed dose) within the range of 80%-120%. In the semaglutide group, there was one subject (1.9%) with compliance <80%.

The relative dose intensity (actual administered dose/planned dose for the dose group) for each treatment group was as follows:
Semaglutide group: 76.938% (SD: 37.5625); the compound (I)-12 mg once every two weeks group: 77.096% (SD: 32.5139); the compound (I)-18 mg once every two weeks group: 56.679% (SD: 38.0158); the compound (I) 24-mg once every two weeks group: 53.472% (SD: 36.5147); the compound (I)-24 mg once weekly group: 54.670% (SD: 36.9173).

**Table 7 Summary of Subject Compliance (SS, N=271)**

| **Item** | | | **Once weekly semaglutide-1 mg group (N=54)** | **Once every two weeks GZR18-12 mg group (N=55)** | **Once every two weeks GZR18-18 mg group (N=53)** | **Once every two weeks GZR18-24 mg group (N=55)** | **Once weekly GZR18-24 mg group (N=54)** |
|---|---|---|---|---|---|---|---|
| | **Complia nce (%)** | Case counts (missing data) | 54 (0) | 55 (0) | 53 (0) | 55 (0) | 54 (0) |
| | | Mean (standard deviation) | 99.062 (6.8028) | 99.827 (1.2842) | 100.000 (0.0000) | 100.000 (0.0000) | 100.197 (0.9896) |
| | | Median (Q1, Q3) | 100.000 (100.000, 100.000) | 100.000 (100.000, 100.000) | 100.000 (100.000, 100.000) | 100.000 (100.000, 100.000) | 100.000 (100.000, 100.000) |
| | | Minimum value, Maximum value | 50.00, 100.00 | 90.48, 100.00 | 100.00, 100.00 | 100.00, 100.00 | 98.31, 104.48 |
| **GZR18 injection/ Semaglut ide injection** | **Complia nce** | Good compliance (80%-120%) | 53 (98.1%) | 55 (100%) | 53 (100%) | 55 (100%) | 54 (100%) |
| | | Poor compliance | 1 (1.9%) | 0 | 0 | 0 | 0 |
| | | <80% | 1 (1.9%) | 0 | 0 | 0 | 0 |
| | | >120% | 0 | 0 | 0 | 0 | 0 |
| | **Relative dose intensity (%)** | Case counts (missing data) | 54 (0) | 55 (0) | 53 (0) | 55 (0) | 54 (0) |
| | | Mean (standard deviation) | 76.938 (37.5625) | 77.096 (32.5139) | 56.679 (38.0158) | 53.472 (36.5147) | 54.670 (36.9173) |
| | | Median (Q1, Q3) | 100.000 (56.579, 100.000) | 95.122 (46.341, 100.000) | 56.180 (46.067, 57.303) | 57.971 (13.043, 81.159) | 59.420 (18.841, 81.159) |
| | | Minimum value, Maximum value | 1.32, 100.00 | 2.44, 110.26 | 1.12, 181.63 | 1.45, 125.45 | 0.91, 125.45 |

| | | | | | | | |
|---|---|---|---|---|---|---|---|
| Notes: (1) Compliance (%) = (Actual dose administered / Prescribed dose) × 100%. (2) Compliance below 80% or above 120% will be defined as poor compliance. (3) Relative dose intensity (%) = (Actual dose administered / Planned dose for the dose group) × 100%. | | | | | | | |

### Example 4: Study of Compound (I) in a Dose Titration Strategy for Type 2 Diabetes

In the completed clinical trial of Example 3, two titration regimens were explored in the once every two weeks groups:
V2.0 Protocol: Titration for the 12 mg/18 mg/24 mg group follows the sequence 3 mg-6 mg-6 mg-9 mg-9 mg-12 mg-12 mg-18 mg-18 mg-24 mg-24 mg;
V3.0 Protocol: Titration for the 12 mg/18 mg/24 mg group follows the sequence 3 mg-6 mg-6 mg-12 mg-12 mg-24 mg-24 mg.

The incidence rates of gastrointestinal adverse reactions by dose of the investigational drug are summarized as follows: for compound (I), the rates in the overall participants at 3 mg, 6 mg, 9 mg, 12 mg, 18 mg, and 24 mg were 45.6%, 53.7%, 45.6%, 62.8%, 42.0%, and 38.3%, respectively. When titrating from 3 mg to 12 mg, the incidence of gastrointestinal adverse events increased with higher doses. When titrating from 18 mg to 24 mg, the incidence of gastrointestinal adverse events decreased with higher doses. Furthermore, the incidence of gastrointestinal adverse events at both 18 mg and 24 mg was lower than at the starting dose (3 mg). This indicates that the 3mg-12mg range established a tolerance phase for subjects. From the above results, it can be seen that gradual dose-escalation better ensures subject safety.

The present invention has been described through the above examples, but it should be understood that the above examples are provided for illustrative purposes only and are not intended to limit the scope of the invention to the described examples. Furthermore, those skilled in the art will appreciate that the present invention is not limited to the above examples. Additional variations and modifications may be made based on the teachings of the present invention, and such variations and modifications fall within the scope of protection claimed herein. The scope of protection of the present invention is defined by the appended claims and their equivalents.

### Sequences:

SEQ ID NO.1:
   GLP-1-(7-37) peptide 
His Ala Glu Gly Thr Phe Thr Ser Asp Val Ser Ser Tyr Leu Glu Gly Gln Ala Ala Lys Glu Phe Ile Ala Trp
Leu Val Lys Gly Arg Gly
SEQ ID NO.2:
   [Gly8, Arg34]GLP-1-(7-37) peptide

His Gly Glu Gly Thr Phe Thr Ser Asp Val Ser Ser Tyr Leu Glu Gly Gln Ala Ala Lys Glu Phe Ile Ala Trp
Leu Val Arg Gly Arg Gly

## Claims

1. A method for treating or preventing metabolic syndrome, wherein the method comprising administering 0.25-100 mg of a compound of formula (I), or a pharmaceutically acceptable salt, amide or ester thereof, to a subject in need thereof, 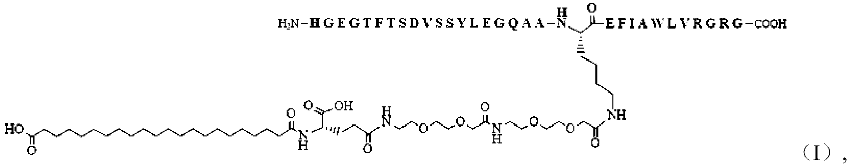
preferably, the metabolic syndrome is diabetes;
preferably, the diabetes is type 2 diabetes.

2. The method according to claim 1, wherein the method comprises administering to the subject in need thereof about 0.25-100 mg, preferably about 0.5-90 mg, preferably about 1-80 mg, preferably about 1.5-80 mg, preferably 2-80 mg, preferably 1.5-70 mg, preferably 2-70 mg, preferably 2.5-70 mg, preferably 3-70 mg, preferably 1.5-60 mg, preferably 3-60 mg, preferably 3.5-60 mg, preferably 1.5-55 mg, preferably 3-55 mg, preferably 1.5-48 mg, preferably 3-48 mg, preferably 3.5-48 mg, preferably 1.5-36 mg, preferably 3-36 mg, preferably 3.5-36 mg, preferably 1.5-24mg, preferably 3-24 mg, preferably 3.5-24 mg, preferably 1.5-18 mg, preferably 3-18mg, preferably 3.5-18 mg, preferably 1.5-12 mg, preferably 1.5-13 mg, preferably 3-12 mg, preferably 3.5-12 mg, preferably 4-60 mg, preferably 5-60 mg, preferably 6-60 mg, preferably 7-60 mg, preferably 8-60 mg, preferably 9-60 mg, preferably 10-60 mg, preferably 11-60 mg, preferably 12-60 mg, preferably 12-48 mg, preferably 12-36 mg, preferably 12-18 mg, preferably 12-24 mg of the compound of formula (I), or the pharmaceutically acceptable salt, amide or ester thereof.

3. The method according to claim 1 or 2, wherein:
the method comprises administering the compound of formula (I), or the pharmaceutically acceptable salt, amide, or ester thereof, to the subject in need thereof at a dosage of 0.25-100 mg , preferably 0.5-90 mg, preferably 1.5-80 mg, preferably 1-80 mg, preferably 1.5-70 mg, preferably 2-80 mg, preferably 2-70 mg, preferably 2.5-70 mg, preferably 3-70 mg, preferably 1.5-60 mg, preferably 3-60 mg, preferably 3.5-60 mg, preferably 1.5-55 mg, preferably 3-55 mg, preferably 1.5-48 mg, preferably 3.5-48 mg, preferably 3-48 mg, preferably 1.5-36 mg, preferably 3-36 mg, preferably 3.5-36 mg, preferably 1.5-24 mg, preferably 3-24 mg, preferably 3.5-24 mg, preferably 1.5-18 mg, preferably 3-18 mg, preferably 3.5-18 mg, preferably 1.5-12 mg, preferably 1.5-13 mg, preferably 3-12 mg, preferably 3.5-12 mg, preferably 4-60 mg, preferably 5-60 mg, preferably 7-60 mg, preferably 8-60 mg, preferably 9-60 mg, preferably 10-60 mg, preferably 11-60 mg, preferably 12-60 mg, preferably 12-48 mg, preferably 12-36 mg, preferably 12-18 mg, preferably 12-24 mg per week or every two weeks.

4. The method according to any one of claims 1 to 3, wherein:
the method comprises administering to the subject in need thereof the compound of formula (I), or the pharmaceutically acceptable salt, amide or ester thereof, at the following doses per week or every two weeks: 0.25 mg, 0.5 mg, 1 mg, 1.5 mg, 2 mg, 2.5 mg, 3 mg, 3.5 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, or 70 mg.

5. The method according to any one of claims 1 to 4, wherein:
the method comprises administering the compound of formula (I), or the pharmaceutically acceptable salt, amide or ester thereof, to the subject in need thereof once weekly or less frequently, preferably once every two weeks or less frequently.

6. The method according to any one of claims 1 to 5, wherein:
the method comprises administering the compound of formula (I), or the pharmaceutically acceptable salt, amide, or ester thereof to the subject in need thereof at the following doses once weekly or once every two weeks: 0.25 mg, 0.5 mg, 1 mg, 1.5 mg, 2 mg, 2.5 mg, 3 mg, 3.5 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, or 70 mg; preferably, the dose of each administration is independently the same or different.

7. The method according to any one of claims 1 to 6, wherein:
the method comprises first administering an escalation dose to the subject in need thereof at a frequency of once weekly or once every two weeks during an escalation dose period, and then administering a maintenance dose at a frequency of once weekly or once every two weeks during a maintenance dose period, wherein
the escalation dose period is at least two weeks, preferably about 2-60 weeks, preferably about 2-55 weeks, preferably about 2-50 weeks, preferably about 2-40 weeks, preferably about 2-31 weeks, preferably about 2-30 weeks, preferably about 2-20 weeks, preferably the escalation dose period is about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, about 52 weeks, about 53 weeks, about 54 weeks, about 55 weeks, about 56 weeks, about 57 weeks, about 58 weeks, about 59 weeks, or about 60 weeks;
the maintenance dose period is at least two weeks, preferably about 3 weeks or more, about 4 weeks or more, about 5 weeks or more, about 6 weeks or more, about 7 weeks or more, about 8 weeks or more, about 9 weeks or more, about 10 weeks or more, about 11 weeks or more, about 12 weeks or more, about 13 weeks or more, about 14 weeks or more, about 15 weeks or more, about 16 weeks or more, about 17 weeks or more, about 18 weeks or more, about 19 weeks or more, about 20 weeks or more, about 21 weeks or more, about 22 weeks or more, about 23 weeks or more, about 24 weeks or more, about 25 weeks or more, about 26 weeks or more, about 27 weeks or more, about 28 weeks or more, about 29 weeks or more, about 30 weeks or more, about 31 weeks or more, about 32 weeks or more, about 33 weeks or more, about 34 weeks or more, about 35 weeks or more, about 36 weeks or more, about 37 weeks or more, about 38 weeks or more, about 39 weeks or more, about 40 weeks or more, about 41 weeks or more, about 42 weeks or more, about 43 weeks or more, about 44 weeks or more, about 45 weeks or more, about 46 weeks or more, about 47 weeks or more, about 48 weeks or more, about 49 weeks or more, about 50 weeks or more, about 51 weeks or more, about 52 weeks or more, about 53 weeks or more, about 54 weeks or more, about 55 weeks or more, about 56 weeks or more, about 57 weeks or more, about 58 weeks or more, about 59 weeks or more, or about 60 weeks or more; preferably about 2-120 weeks, preferably about 2-100 weeks, preferably about 2-80 weeks, preferably about 2-70 weeks, preferably about 2-60 weeks, preferably about 2-55 weeks, preferably about 2-50 weeks, preferably about 2-40 weeks, preferably about 2-35 weeks, preferably about 2-30 weeks, preferably about 2-20 weeks; preferably the maintenance dose period is preferably about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, about 52 weeks, about 53 weeks, about 54 weeks, about 55 weeks, about 56 weeks, about 57 weeks, about 58 weeks, about 59 weeks, or about 60 weeks;
the dose at each administration during the dose escalation is independently about 0.25mg-70mg, preferably about 1.5mg-60mg, preferably about 1.5mg-48mg, preferably about 1.5mg-36mg, preferably about 1.5mg-24mg, preferably about 1.5mg-24mg, preferably about 1.5mg-18mg, preferably about 1.5mg-13mg, preferably about 1.5mg-12mg of the compound of formula (I), or the pharmaceutically acceptable salt, amide or ester thereof; preferably, each administered dose in the escalation dose is independently about 0.25mg, about 0.5mg, 1mg, about 1.5mg, about 2mg, about 2.5mg, about 3mg, about 3.5mg, about 4mg, about 5mg, about 6mg, about 7mg, about 8mg, about 9mg, 10mg, 11mg, about 12mg, 13mg, 14mg, 15mg, 16 mg, 17 mg, about 18 mg, 19 mg, 20 mg, 21 mg, 22 mg, 23 mg, about 24 mg, 25 mg, 26 mg, 27 mg, 28 mg, 29 mg, about 30 mg, 31 mg, 32 mg, 33 mg, 34 mg, 35 mg, about 36 mg, 37 mg, 38 mg, 39 mg, 40 mg, 41 mg, 42 mg, 43 mg, 44 mg, 45 mg, 46 mg, 47 mg, about 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, or 70 mg of the compound of formula (I), or the pharmaceutically acceptable salt, amide or ester thereof;
the maintenance dose is about 3-70 mg, preferably about 6 mg-60 mg, preferably about 9 mg-48 mg, preferably about 12 mg-48 mg, preferably about 12 mg-36 mg, preferably about 12 mg-24 mg, preferably about 12 mg to 18 mg, preferably about 13 mg to 24 mg, preferably about 13 mg to 36 mg of the compound of formula (I), or the pharmaceutically acceptable salt, amide or ester thereof; preferably, the maintenance dose is about 3 mg, 4 mg, 5 mg, 6 mg, 7 mg, 8 mg, about 9 mg, 10 mg, 11 mg, about 12 mg, 13 mg, 14 mg, 15 mg, 16 mg, 17 mg, about 18 mg, 19 mg, 20 mg, 21 mg, 22 mg, 23 mg, about 24 mg, 25 mg, 26 mg, 27 mg, 28 mg, 29 mg, 30 mg, 31 mg, 32 mg 67mg, 68mg, 69mg, or 70mg of the compound of formula (I), or the pharmaceutically acceptable salt, amide or ester thereof; and
the maintenance dose is greater than or equal to the escalation dose;
preferably:
during the escalation dose period, the administration period of each different escalation dose is independently about 1 week or more, about 2 weeks or more, about 3 weeks or more, about 4 weeks or more, about 5 weeks or more, about 6 weeks or more, about 7 weeks or more, about 8 weeks or more, about 9 weeks or more, about 10 weeks or more, about 11 weeks or more, about 12 weeks or more, about 13 weeks or more, about 14 weeks or more, about 15 weeks or more, about 16 weeks or more, about 17 weeks or more, about 18 weeks or more, about 19 weeks or more, or about 20 weeks or more; preferably, during the escalation dose period, the administration period of each different escalation dose is independently about 1 week, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, or about 20 weeks;
preferably:
during the escalation dose period, the escalation dose is about 1.1-3 times of the preceding different escalation dose, preferably about 1.1-2 times, preferably about 1.1 times, about 1.2 times, about 1.3 times, about 1.4 times, about 1.5 times, about 1.6 times, about 1.7 times, about 1.8 times, about 1.9 times, about 2.1 times, about 2.2 times, about 2.3 times, about 2.4 times, about 2.5 times, about 2.6 times, about 2.7 times, about 2.8 times, about 2.9 times or about 3 times.

8. The method according to claim 7, wherein:
the escalation dose period is about 6-31 weeks, 8-31 weeks, preferably about 10-31 weeks, preferably about 10-24 weeks, preferably about 14-20 weeks, preferably about 16-20 weeks, preferably the escalation dose period is about 6 weeks, 8 weeks, 10 weeks, about 12 weeks, about 14 weeks, about 16 weeks, about 18 weeks, about 20 weeks, about 30 weeks or about 31 weeks; preferably the escalation dose period is about 10 weeks, about 14 weeks, about 16 weeks, about 18 weeks, about 20 weeks, or about 31 weeks;
the maintenance dose period is about 4 weeks or more, 5 weeks or more, about 12 weeks or more, about 14 weeks or more, about 16 weeks or more, about 18 weeks or more, about 20 weeks or more, about 30 weeks or more, about 40 weeks or more, about 50 weeks or more, about 60 weeks or more, about 70 weeks or more, about 80 weeks or more, or about 90 weeks or more;
preferably the maintenance dose period is about 4-36 weeks; preferably, the maintenance dose period is about 4 weeks, about 5 weeks, about 10 weeks, about 12 weeks, about 14 weeks, about 16 weeks, about 18 weeks, about 20 weeks, about 22 weeks, about 24 weeks, about 26 weeks, about 28 weeks, about 30 weeks, about 32 weeks, about 34 weeks or about 36 weeks; preferably, the maintenance dose period is about 4-30 weeks, preferably, the maintenance dose period is about 4 weeks, about 5 weeks, about 10 weeks, about 12 weeks, about 14 weeks, about 16 weeks, about 18 weeks, about 20 weeks, about 22 weeks, about 24 weeks, about 26 weeks, or about 30 weeks;
the dose at each administration during the dose escalation is independently about 0.5 mg, 1 mg, 1.5 mg, 2 mg, 2.5 mg, 3 mg, 3.5 mg, 4 mg, 5 mg, 6 mg, 7 mg, 9 mg, 11 mg, 12 mg, 13 mg, 15 mg, 18 mg, 24 mg, 30 mg, 36 mg, or 48 mg of the compound of formula (I), or the pharmaceutically acceptable salt, amide or ester thereof; preferably, each administered dose in the escalation dose is independently about 1.5 mg, about 3 mg, about 6 mg, about 9 mg, about 12 mg, about 18 mg, about 24 mg, or about 36 mg of the compound of formula (I), or the pharmaceutically acceptable salt, amide or ester thereof; or each administered dose in the escalation dose is independently is about 1.5 mg, about 3 mg, about 5 mg, about 7 mg, about 9 mg, about 11 mg, about 13 mg or about 15 mg of the compound of formula (I), or the pharmaceutically acceptable salt, amide or ester thereof;
the maintenance dose is about 6 mg, about 12 mg, about 13 mg, about 18 mg, about 24 mg, about 30 mg, about 36 mg, or about 48 mg of the compound of formula (I), or the pharmaceutically acceptable salt, amide or ester thereof; and
the maintenance dose is greater than or equal to the escalation dose.

9. The method according to claim 7 or 8, wherein:
the method comprises first administering the escalation dose to the subject in need thereof at a frequency of once weekly or once every two weeks during the escalation dose period, and then administering the maintenance dose at a frequency of once weekly or once every two weeks during the maintenance dose period, wherein,
the escalation dose period is about 8 weeks, 10 weeks, about 14 weeks, about 16 weeks, about 18 weeks, about 20 weeks, about 30 weeks, or about 31 weeks;
the maintenance dose period is about 4 weeks or more, about 12 weeks or more, about 16 weeks or more, about 20 weeks or more, about 30 weeks or more, about 40 weeks or more, about 50 weeks or more, about 60 weeks or more, about 70 weeks or more, about 80 weeks or more, or about 90 weeks or more; preferably, the maintenance dose period is about 4 weeks, about 5 weeks, about 10 weeks, about 12 weeks, about 14 weeks, about 16 weeks, about 18 weeks, about 20 weeks, about 22 weeks, about 24 weeks, about 26 weeks, about 28 weeks, about 30 weeks, about 32 weeks, about 34 weeks or about 36 weeks; preferably, the maintenance dose period is about 4 weeks, about 12 weeks, about 16 weeks, about 20 weeks, about 24 weeks, 26 weeks, 28 weeks, or about 30 weeks;
the dose at each administration during the dose escalation is independently about 1.5 mg, 3 mg, 5 mg, 6 mg, 7 mg, 9 mg, 11 mg, 12 mg, 13 mg, 15 mg, 18 mg, 24 mg, 30 mg, or 36 mg of the compound of formula (I) or the pharmaceutically acceptable salt, amide or ester thereof;
the maintenance dose is about 3 mg, 6 mg, 12 mg, 13 mg, 18 mg, 24 mg, 30 mg, 36 mg, or 48 mg of the compound of formula (I), or the pharmaceutically acceptable salt, amide or ester thereof; and
the maintenance dose is greater than or equal to the escalation dose;
preferably:
when the maintenance dose is about 12 mg of the compound of formula (I), or the pharmaceutically acceptable salt, amide or ester thereof, the method comprises sequentially administering to the subject in need thereof 1.5 mg of the compound of formula (I), or the pharmaceutically acceptable salt, amide or ester thereof, once every two weeks for 2 weeks or more or 4 weeks or more; administering 3 mg of the compound of formula (I), or the pharmaceutically acceptable salt, amide or ester thereof, once every two weeks for 2 weeks or more or 4 weeks or more; administering 6 mg of the compound of formula (I), or the pharmaceutically acceptable salt, amide or ester thereof once every two weeks for 4 weeks or more; administering 9 mg of the compound of formula (I), or the pharmaceutically acceptable salt, amide or ester thereof once every two weeks for 4 weeks or more; and administering 12 mg of the compound of formula (I), or the pharmaceutically acceptable salt, amide or ester thereof once every two weeks for 20 weeks or more, preferably about 25 weeks or more, preferably about 30 weeks or more, preferably about 20-120 weeks, preferably about 20-100 weeks, preferably about 20-80 weeks; preferably about 20-60 weeks; or
when the maintenance dose is about 18 mg of the compound of formula (I), or the pharmaceutically acceptable salt, amide or ester thereof, the method comprises sequentially administering to the subject in need thereof 1.5 mg of the compound of formula (I), or the pharmaceutically acceptable salt, amide or ester thereof, once every two weeks for 2 weeks or more or 4 weeks or more; administering 3 mg of the compound of formula (I), or the pharmaceutically acceptable salt, amide or ester thereof, once every two weeks for 2 weeks or more or 4 weeks or more; administering 6 mg of the compound of formula (I), or the pharmaceutically acceptable salt, amide or ester thereof, once every two weeks for 4 weeks or more; administering 9 mg of the compound of formula (I), or the pharmaceutically acceptable salt, amide or ester thereof, once every two weeks for 4 weeks or more; administering 12 mg of the compound of formula (I), or the pharmaceutically acceptable salt, amide or ester thereof at a frequency of once every two weeks for 4 weeks or more; and administering 18 mg of the compound of formula (I), or the pharmaceutically acceptable salt, amide or ester thereof at a frequency of once every two weeks for 16 weeks or more, preferably about 18 weeks or more, preferably about 20 weeks or more, preferably about 25 weeks or more, preferably about 30 weeks or more, preferably about 20-120 weeks, preferably about 20-100 weeks, preferably about 20-80 weeks; preferably about 20-60 weeks; or
when the maintenance dose is about 12 mg of the compound of formula (I), or the pharmaceutically acceptable salt, amide or ester thereof, the method comprises sequentially administering to the subject in need thereof 3 mg of the compound of formula (I), or the pharmaceutically acceptable salt, amide or ester thereof, once every two weeks for 2 weeks or more or 4 weeks or more; administering 6 mg of the compound of formula (I), or the pharmaceutically acceptable salt, amide or ester thereof, once every two weeks for 4 weeks or more; administering 12 mg of the compound of formula (I), or the pharmaceutically acceptable salt, amide or ester thereof, once every two weeks for 18 weeks or more, preferably about 20 weeks or more, preferably about 25 weeks or more, preferably about 30 weeks or more, preferably about 20-120 weeks, preferably about 20-100 weeks, preferably about 20-80 weeks; preferably about 20-60 weeks; or
when the maintenance dose is about 12 mg of the compound of formula (I), or the pharmaceutically acceptable salt, amide or ester thereof, the method comprises sequentially administering to the subject in need thereof 1.5 mg of the compound of formula (I), or the pharmaceutically acceptable salt, amide or ester thereof, once every two weeks for 4 weeks or more; administering 3 mg of the compound of formula (I), or the pharmaceutically acceptable salt, amide or ester thereof, once every two weeks for 4 weeks or more; administering 6 mg of the compound of formula (I), or the pharmaceutically acceptable salt, amide or ester thereof, once every two weeks for 4 weeks or more; administering 9 mg of the compound of formula (I), or the pharmaceutically acceptable salt, amide or ester thereof, once every two weeks for 4 weeks or more; and administering 12 mg of the compound of formula (I), or the pharmaceutically acceptable salt, amide or ester thereof, once every two weeks for 28 weeks or more or 36 weeks or more; preferably about 14 weeks or more, preferably about 16 weeks or more, preferably about 18 weeks or more, preferably about 20 weeks or more, preferably about 25 weeks or more, preferably about 30 weeks or more, preferably about 20-120 weeks, preferably about 20-100 weeks, preferably about 20-80 weeks; preferably about 20-60 weeks; or
when the maintenance dose is about 18 mg of the compound of formula (I), or the pharmaceutically acceptable salt, amide or ester thereof, the method comprises sequentially administering to the subject in need thereof 1.5 mg of the compound of formula (I), or the pharmaceutically acceptable salt, amide or ester thereof, once every two weeks for 4 weeks or more; administering 3 mg of the compound of formula (I), or the pharmaceutically acceptable salt, amide or ester thereof, once every two weeks for 4 weeks or more; administering 6 mg of the compound of formula (I), or the pharmaceutically acceptable salt, amide or ester thereof, once every two weeks for 4 weeks or more; administering 9 mg of the compound of formula (I), or the pharmaceutically acceptable salt, amide or ester thereof, once every two weeks for 4 weeks or more, administering 12 mg of the compound of formula (I), or the pharmaceutically acceptable salt, amide or ester thereof, once every two weeks for 4 weeks or more; and administering 18 mg of the compound of formula (I), or the pharmaceutically acceptable salt, amide or ester thereof, once every two weeks for 24 weeks or more or 32 weeks or more, preferably about 14 weeks or more, preferably about 16 weeks or more, preferably about 18 weeks or more, preferably about 20 weeks or more, preferably about 25 weeks or more, preferably about 30 weeks or more, preferably about 20-120 weeks, preferably about 20-100 weeks, preferably about 20-80 weeks; preferably about 20-60 weeks; or
when the maintenance dose is about 15 mg, 17 mg, 18 mg, 19 mg, 21 mg, 24 mg or 36 mg of the compound of formula (I), or the pharmaceutically acceptable salt, amide or ester thereof, the method comprises sequentially administering to the subject in need thereof 1.5 mg of the compound of formula (I), or the pharmaceutically acceptable salt, amide or ester thereof, once weekly for one week or more; administering 3 mg of the compound of formula (I), or the pharmaceutically acceptable salt, amide or ester thereof, once weekly for 2 weeks or more; administering 5 mg of the compound of formula (I), or the pharmaceutically acceptable salt, amide or ester thereof, once weekly for 2 weeks or more; administering 7 mg of the compound of formula (I), or the pharmaceutically acceptable salt, amide or ester thereof, once weekly for 4 weeks or more; administering 9 mg of the compound of formula (I), or the pharmaceutically acceptable salt, amide or ester thereof, once weekly for 4 weeks or more; administering 11 mg of the compound of formula (I), or the pharmaceutically acceptable salt, amide or ester thereof, once weekly for 4 weeks or more; administering 13 mg of the compound of formula (I), or the pharmaceutically acceptable salt, amide or ester thereof, once weekly for 4 weeks or more; or
when the maintenance dose is about 24 mg of the compound of formula (I), or the pharmaceutically acceptable salt, amide or ester thereof, the method comprises sequentially administering to the subject in need thereof 3 mg of the compound of formula (I), or the pharmaceutically acceptable salt, amide or ester thereof, once every two weeks for 2 weeks or more; administering 6 mg of the compound of formula (I), or the pharmaceutically acceptable salt, amide or ester thereof, once every two weeks for 4 weeks or more; administering 12 mg of the compound of formula (I), or the pharmaceutically acceptable salt, amide or ester thereof, once every two weeks for 4 weeks or more; administering 24 mg of the compound of formula (I), or the pharmaceutically acceptable salt, amide or ester thereof, once every two weeks for 14 weeks or more, preferably about 16 weeks or more, preferably about 18 weeks or more, preferably about 20 weeks or more, preferably about 25 weeks or more, preferably about 30 weeks or more, preferably about 20-120 weeks, preferably about 20-100 weeks, preferably about 20-80 weeks; preferably about 20-60 weeks; or
when the maintenance dose is about 48 mg of the compound of formula (I), or the pharmaceutically acceptable salt, amide or ester thereof, the method comprises sequentially administering to the subject in need thereof 3 mg of the compound of formula (I), or the pharmaceutically acceptable salt, amide or ester thereof, once every two weeks for 2 weeks or more; administering 6 mg of the compound of formula (I), or the pharmaceutically acceptable salt, amide or ester thereof, once every two weeks for 4 weeks or more; administering 12 mg of the compound of formula (I), or the pharmaceutically acceptable salt, amide or ester thereof, once every two weeks for 4 weeks or more; administering 24 mg of the compound of formula (I), or the pharmaceutically acceptable salt, amide or ester thereof, once every two weeks for 4 weeks or more; administering 36 mg of the compound of formula (I), or the pharmaceutically acceptable salt, amide or ester thereof, once every two weeks for 4 weeks or more; administering 48 mg of the compound of formula (I), or the pharmaceutically acceptable salt, amide or ester thereof, once every two weeks for 12 weeks or more; or
when the maintenance dose is about 36 mg of the compound of formula (I), or the pharmaceutically acceptable salt, amide or ester thereof, the method comprises sequentially administering to the subject in need thereof 3 mg of the compound of formula (I), or the pharmaceutically acceptable salt, amide or ester thereof, once every two weeks for 2 weeks or more; administering 6 mg of the compound of formula (I), or the pharmaceutically acceptable salt, amide or ester thereof, once every two weeks for 4 weeks or more; administering 12 mg of the compound of formula (I), or the pharmaceutically acceptable salt, amide or ester thereof, once every two weeks for 4 weeks or more; administering 24 mg of the compound of formula (I), or the pharmaceutically acceptable salt, amide or ester thereof, once every two weeks for 4 weeks or more; and administering 36 mg of the compound of formula (I), or the pharmaceutically acceptable salt, amide or ester thereof, once every two weeks for 10 weeks or more.

10. The method according to any one of claims 1 to 9, wherein:
the method comprises administering to the subject in need thereof a first dose of 0.25-5 mg of the compound of formula (I), or the pharmaceutically acceptable salt, amide or ester thereof, once weekly or once every two weeks;
after at least one week of administration of the first dose, administering a second dose of 3-12 mg of the compound of formula (I), or the pharmaceutically acceptable salt, amide or ester thereof, once weekly or once every two weeks; preferably, after 1, 2, 3 or 4 weeks of administration of the first dose, administering the second dose of 3-12 mg of the compound of formula (I), or the pharmaceutically acceptable salt, amide or ester thereof, once weekly or once every two weeks;
after the second dose is administered for 2, 3, 4, 5, 6, 7, or 8 weeks, administering a third dose of 6-24 mg of the compound of formula (I), or the pharmaceutically acceptable salt, amide or ester thereof, once weekly or once every two weeks.

11. The method according to claim 10, wherein:
after administering the third dose for 2, 3, 4, 5, 6, 7, 8, 9, 10, 11, 12, 13, 14, 15, 16, 17, 18, 19, or 20 weeks, administering a fourth dose of 9-48 mg of the compound of formula (I) or the pharmaceutically acceptable salt, amide or ester thereof once weekly or once every two weeks.

12. The method according to claim 11, wherein:
after administering the fourth dose for 2, 3, 4, 5, 6, 7, 8, 9, 10, 11, 12, 13, 14, 15, 16, 17, 18, 19, or 20 weeks, administering a fifth dose of 12-60 mg of the compound of formula (I) or the pharmaceutically acceptable salt, amide or ester thereof once weekly or once every two weeks.

13. The method according to claim 12, wherein:
after administering the fifth dose for 2, 3, 4, 5, 6, 7, 8, 9, 10, 11, 12, 13, 14, 15, 16, 17, 18, 19, or 20 weeks, administering a sixth dose of 18-72 mg of the compound of formula (I) or the pharmaceutically acceptable salt, amide or ester thereof once weekly or once every two weeks.

14. The method according to claim 13, wherein:
after administering the sixth dose for 2, 3, 4, 5, 6, 7, 8, 9, 10, 11, 12, 13, 14, 15, 16, 17, 18, 19, or 20 weeks, administering a seventh dose of 24-75 mg of the compound of formula (I) or the pharmaceutically acceptable salt, amide or ester thereof once weekly or once every two weeks.

15. The method according to any one of claims 10 to 14, wherein:
the first dose is 0.5-5 mg, preferably 1-4.5 mg, preferably 1.5-4 mg, preferably 1.5-3.5 mg, preferably 1.5-3.0 mg, preferably 2-3 mg, preferably 1.5 mg or 3 mg; and/or
the second dose is 3-12 mg, preferably 3.5-7.5 mg, preferably 4-7 mg, preferably 4.5-6.5 mg, preferably 5-6 mg, preferably 3 mg or 6 mg; and/or
the third dose is 6-20 mg, preferably 6-18 mg, preferably 6-12 mg, preferably 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, preferably 6 mg or 12 mg; and/or
the fourth dose is 9-36 mg, preferably 12-36 mg, preferably 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, or 36 mg, preferably 9 mg or 24 mg; and/or
the fifth dose is 12-48 mg, preferably 18-48 mg, preferably 18-36 mg, preferably 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg or 48 mg, preferably 12 mg or 36 mg; and/or
the sixth dose is 18-60 mg, preferably 24-60 mg, preferably 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, or 60 mg, preferably 18 mg or 48 mg; and/or
the seventh dose is 24-70 mg, preferably 24-60 mg, preferably 24-48 mg, preferably 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, or 70 mg, preferably 24 mg or 48 mg.

16. The method according to any one of claims 1-6 or 7-9 or 10-15, wherein:
the method comprises administering a first dose of 3 mg of the compound of formula (I), or the pharmaceutically acceptable salt, amide or ester thereof, to the subject in need thereof at a frequency of once weekly or once every two weeks;
after administering the first dose for 2 or 4 weeks, administering a second dose of 6 mg of the compound of formula (I), or the pharmaceutically acceptable salt, amide or ester thereof, once weekly or once every two weeks; and
after the second dose is administered for 4 weeks, administering a third dose of 12 mg of the compound of formula (I), or the pharmaceutically acceptable salt, amide or ester thereof, once weekly or once every two weeks; preferably, administering the third dose of 12 mg of the compound of formula (I), or the pharmaceutically acceptable salt, amide or ester thereof, once weekly or once every two weeks for 2 weeks or more, preferably 4 weeks or more, preferably 8 weeks or more, preferably 12 weeks or more, preferably 16 weeks or more, preferably 18 weeks or more, preferably 20 weeks or more; preferably, administering the third dose of 12 mg of the compound of formula (I), or the pharmaceutically acceptable salt, amide or ester thereof, once weekly or once every two weeks for 18 weeks.

17. The method according to any one of claims 1-6, 7-9 or 10-15, wherein:
the method comprises administering a first dose of 3 mg of the compound of formula (I), or the pharmaceutically acceptable salt, amide or ester thereof, to the subject in need thereof once weekly or once every two weeks;
after administering the first dose for 2 or 4 weeks, administering a second dose of 6 mg of the compound of formula (I), or the pharmaceutically acceptable salt, amide or ester thereof, once weekly or once every two weeks;
after administering the second dose for 4 weeks, administering a third dose of 12 mg of the compound of formula (I), or the pharmaceutically acceptable salt, amide or ester thereof, once weekly or once every two weeks; and
after administering the third dose for 4 weeks, administering a fourth dose of 24 mg of the compound of formula (I) or the pharmaceutically acceptable salt, amide or ester thereof once weekly or once every two weeks; preferably, administering the fourth dose of 24 mg of the compound of formula (I) or the pharmaceutically acceptable salt, amide or ester thereof once weekly or once every two weeks for 2 weeks or more, preferably 4 weeks or more, preferably 8 weeks or more, preferably 12 weeks or more, preferably 14 weeks or more, preferably 16 weeks or more, preferably 20 weeks or more; preferably, administering the fourth dose of 24 mg of the compound of formula (I) or the pharmaceutically acceptable salt, amide or ester thereof once weekly or once every two weeks for 14 weeks.

18. The method according to any one of claims 1-6, 7-9 or 10-15, wherein:
the method comprises administering a first dose of 3 mg of the compound of formula (I), or the pharmaceutically acceptable salt, amide or ester thereof, to the subject in need thereof once weekly or once every two weeks;
after administering the first dose for 2 or 4 weeks, administering a second dose of 6 mg of the compound of formula (I), or the pharmaceutically acceptable salt, amide or ester thereof, once weekly or once every two weeks;
after administering the second dose for 4 weeks, administering a third dose of 12 mg of the compound of formula (I), or the pharmaceutically acceptable salt, amide or ester thereof, once weekly or once every two weeks; and
after administering the third dose for 4 weeks, administering a fourth dose of 24 mg of the compound of formula (I) or the pharmaceutically acceptable salt, amide or ester thereof once weekly or once every two weeks; and
after administering the fourth dose for 4 weeks, administering a fifth dose of 36 mg of the compound of formula (I) or the pharmaceutically acceptable salt, amide or ester thereof once weekly or once every two weeks; preferably, administering the fifth dose of 36 mg of the compound of formula (I) or the pharmaceutically acceptable salt, amide or ester thereof once weekly or once every two weeks for 2 weeks or more, preferably 4 weeks or more, preferably 8 weeks or more, preferably 10 weeks or more, preferably 12 weeks or more, preferably 14 weeks or more, preferably 16 weeks or more, preferably 20 weeks or more; preferably, administering the fifth dose of 36 mg of the compound of formula (I) or the pharmaceutically acceptable salt, amide or ester thereof once weekly or once every two weeks for 10 weeks.

19. The method according to any one of claims 1-6, 7-9 or 10-15, wherein:
the method comprises administering a first dose of 3 mg of the compound of formula (I), or the pharmaceutically acceptable salt, amide or ester thereof, to the subject in need thereof at a frequency of once weekly or once every two weeks;
after administering the first dose for 2 or 4 weeks, administering a second dose of 6 mg of the compound of formula (I), or the pharmaceutically acceptable salt, amide or ester thereof, once weekly or once every two weeks;
after administering the second dose for 4 weeks, administering a third dose of 12 mg of the compound of formula (I), or the pharmaceutically acceptable salt, amide or ester thereof, once weekly or once every two weeks;
after administering the third dose for 4 weeks, administering a fourth dose of 24 mg of the compound of formula (I) or the pharmaceutically acceptable salt, amide or ester thereof at a frequency of once weekly or once every two weeks;
after administering the fourth dose for 4 weeks, administering a fifth dose of 36 mg of the compound of formula (I) or the pharmaceutically acceptable salt, amide or ester thereof once weekly or once every two weeks; and
after administering the fifth dose for 4 weeks, administering a sixth dose of 48 mg of the compound of formula (I) or the pharmaceutically acceptable salt, amide or ester thereof once weekly or once every two weeks; preferably, administering the sixth dose of 48 mg of the compound of formula (I) or the pharmaceutically acceptable salt, amide or ester thereof once weekly or once every two weeks for 2 weeks or more, preferably 4 weeks or more, preferably 8 weeks or more, preferably 12 weeks or more, preferably 16 weeks or more, preferably 20 weeks or more; preferably, administering the sixth dose of 48 mg of the compound of formula (I) or the pharmaceutically acceptable salt, amide or ester thereof once weekly or once every two weeks for 12 weeks.

20. The method according to any one of claims 1-6, 7-9 or 10-15, wherein:
the method comprises administering to the subject in need thereof a first dose of 1.5 mg of a compound of formula (I), or the pharmaceutically acceptable salt, amide or ester thereof, once weekly or once every two weeks;
after administering the first dose for 2 or 4 weeks, administering a second dose of 3 mg of the compound of formula (I), or the pharmaceutically acceptable salt, amide or ester thereof, once weekly or once every two weeks;
after administering the second dose for 4 weeks, administering a third dose of 6 mg of the compound of formula (I), or the pharmaceutically acceptable salt, amide or ester thereof, once weekly or once every two weeks;
after administering the third dose for 4 weeks, administering a fourth dose of 9 mg of the compound of formula (I) or the pharmaceutically acceptable salt, amide or ester thereof once weekly or once every two weeks;
after administering the fourth dose for 4 weeks, administering a fifth dose of 12 mg of the compound of formula (I) or the pharmaceutically acceptable salt, amide or ester thereof once weekly or once every two weeks; preferably, administering the fifth dose of 12 mg of the compound of formula (I) or the pharmaceutically acceptable salt, amide or ester thereof once weekly or once every two weeks for 2 weeks or more, preferably 4 weeks or more, preferably 8 weeks or more, preferably 12 weeks or more, preferably 16 weeks or more, preferably 20 weeks or more; preferably, administering the fifth dose of 12 mg of the compound of formula (I) or the pharmaceutically acceptable salt, amide or ester thereof once weekly or once every two weeks for 8 weeks or more; preferably, administering 12 mg of the compound of formula (I), or the pharmaceutically acceptable salt, amide or ester thereof once every two weeks for 28 weeks or more or 36 weeks or more.

21. The method according to any one of claims 1-6, 7-9 or 10-15, wherein:
the method comprises administering to the subject in need thereof a first dose of 1.5 mg of a compound of formula (I), or the pharmaceutically acceptable salt, amide or ester thereof, once weekly or once every two weeks;
after administering the first dose for 4 weeks, administering a second dose of 3 mg of the compound of formula (I), or the pharmaceutically acceptable salt, amide or ester thereof, once weekly or once every two weeks;
after administering the second dose for 4 weeks, administering a third dose of 6 mg of the compound of formula (I), or the pharmaceutically acceptable salt, amide or ester thereof once weekly or once every two weeks;
after administering the third dose for 4 weeks, administering a fourth dose of 9 mg of the compound of formula (I), or the pharmaceutically acceptable salt, amide or ester thereof, once weekly or once every two weeks;
after administering the fourth dose for 4 weeks, administering a fifth dose of 12 mg of the compound of formula (I) or the pharmaceutically acceptable salt, amide or ester thereof once weekly or once every two weeks;
after administering the fifth dose for 4 weeks, administering a sixth dose of 18 mg of the compound of formula (I) or the pharmaceutically acceptable salt, amide or ester thereof once weekly or once every two weeks, preferably administering the sixth dose of 18 mg of the compound of formula (I) or the pharmaceutically acceptable salt, amide or ester thereof once weekly or once every two weeks for 2 weeks or more, preferably 4 weeks or more, preferably 8 weeks or more, preferably 12 weeks or more, preferably 16 weeks or more, preferably 20 weeks or more; preferably administering the sixth dose of 18 mg of the compound of formula (I) or the pharmaceutically acceptable salt, amide or ester thereof once weekly or once every two weeks for 4 weeks; preferably administering 18 mg of a compound of formula (I), or the pharmaceutically acceptable salt, amide or ester thereof, once every two weeks for 24 weeks or more or 32 weeks or more.

22. The method according to any one of claims 1-6, 7-9 or 10-15, wherein:
the method comprises administering to the subject in need thereof a first dose of about 1.5 mg of the compound of formula (I), or the pharmaceutically acceptable salt, amide or ester thereof, once weekly or once every two weeks;
after administering the first dose for 4 weeks, administering a second dose of about 3 mg of the compound of formula (I), or the pharmaceutically acceptable salt, amide or ester thereof, once weekly or once every two weeks;
after administering the second dose for 4 weeks, administering a third dose of about 6 mg of the compound of formula (I), or the pharmaceutically acceptable salt, amide or ester thereof, once weekly or once every two weeks;
after administering the third dose for 4 weeks, administering a fourth dose of about 9 mg of the compound of formula (I), or the pharmaceutically acceptable salt, amide or ester thereof, once weekly or once every two weeks;
after administering the fourth dose for 4 weeks, administering a fifth dose of about 12 mg of the compound of formula (I) or the pharmaceutically acceptable salt, amide or ester thereof once weekly or once every two weeks;
after administering the fifth dose for 4 weeks, administering a sixth dose of about 18 mg of the compound of formula (I) or the pharmaceutically acceptable salt, amide or ester thereof once weekly or once every two weeks;
after administering the sixth dose for 4 weeks, administering a seventh dose of about 24 mg of the compound of formula (I) or the pharmaceutically acceptable salt, amide or ester thereof once weekly or once every two weeks; preferably, administering the seventh dose of 24 mg of the compound of formula (I) or the pharmaceutically acceptable salt, amide or ester thereof once weekly or once every two weeks for 2 weeks or more, preferably 4 weeks or more, preferably 8 weeks or more, preferably 12 weeks or more, preferably 16 weeks or more, preferably 20 weeks or more; preferably, administering the seventh dose of 24 mg of the compound of formula (I) or the pharmaceutically acceptable salt, amide or ester thereof once weekly or once every two weeks for 4 weeks.

23. The method according to any one of claims 1 to 6 or 7 to 9, wherein:
the method comprises administering to the subject in need thereof a first' dose of 0.5-1.5 mg of the compound of formula (I), or the pharmaceutically acceptable salt, amide or ester thereof, once weekly or once every two weeks; and/or
after administering the first' dose for 1, 2, 3 or 4 weeks, administering a second' dose of 1-3 mg of the compound of formula (I), or the pharmaceutically acceptable salt, amide or ester thereof, once weekly or once every two weeks; and/or
after administering the second' dose for 2, 3, 4, 5, 6, 7, or 8 weeks, administering a third' dose of 2-6 mg of the compound of formula (I), or the pharmaceutically acceptable salt, amide or ester thereof, once weekly or once every two weeks; and/or
after administering the third' dose for 2, 3, 4, 5, 6, 7, or 8 weeks, administering a fourth' dose of 2.5-9 mg of the compound of formula (I), or the pharmaceutically acceptable salt, amide or ester thereof, once weekly or once every two weeks; and/or
after administering the fourth' dose for 2, 3, 4, 5, 6, 7, 8, 9, 10, 11, 12, 13, 14, 15, 16, 17, 18, 19, or 20 weeks, administering a fifth' dose of 3-12 mg of the compound of formula (I) or the pharmaceutically acceptable salt, amide or ester thereof once weekly or once every two weeks; and/or
after administering the fifth' dose for 2, 3, 4, 5, 6, 7, 8, 9, 10, 11, 12, 13, 14, 15, 16, 17, 18, 19, or 20 weeks, administering a sixth' dose of 3.5-18 mg of the compound of formula (I) or the pharmaceutically acceptable salt, amide or ester thereof once weekly or once every two weeks; and/or
after administering the sixth' dose for 2, 3, 4, 5, 6, 7, 8, 9, 10, 11, 12, 13, 14, 15, 16, 17, 18, 19, or 20 weeks, administering a seventh' dose of 4-24 mg of the compound of formula (I) or the pharmaceutically acceptable salt, amide or ester thereof once weekly or once every two weeks; and/or
after administering the seventh' dose for 2, 3, 4, 5, 6, 7, 8, 9, 10, 11, 12, 13, 14, 15, 16, 17, 18, 19, or 20 weeks, administering an eighth' dose of 6-30 mg of the compound of formula (I) or the pharmaceutically acceptable salt, amide or ester thereof once weekly or once every two weeks.

24. The method according to any one of claims 1-6, 7-9 or 23, wherein:
the first' dose is 0.5-1.5 mg, preferably 0.5 mg, 0.6 mg, 1 mg, 1.2 mg, 1.5 mg, preferably 0.5 mg or 1.5 mg; and/or
the second'e dose is 1-3 mg, preferably 1 mg, 1.5 mg, 2 mg, 2.5 mg, 3 mg, preferably 1 mg or 3 mg; and/or
the third' dose is 2-6 mg, preferably 2 mg, 3 mg, 4 mg, 5 mg, 6 mg, preferably 5 mg or 6 mg; and/or
the fourth' dose is 2.5-9 mg, preferably 2.5 mg, 3 mg, 4 mg, 5 mg, 6 mg, 7 mg, 8 mg, 9 mg, preferably 2.5 mg, 7 mg or 9 mg; and/or
the fifth' dose is 3-12 mg, preferably 7 mg, 8 mg, 9 mg, 10 mg, 11 mg, or 12 mg, preferably 3 mg, 9 mg or 12 mg; and/or
the sixth' dose is 3.5-18 mg, preferably 3.5 mg, 4 mg, 5 mg, 6 mg, 7 mg, 8 mg, 9 mg, 10 mg, 11 mg, 12 mg, 13 mg, 14 mg, 15 mg, 16 mg, 17 mg, or 18 mg, preferably 3.5 mg, 11 mg, or 18 mg; and/or
the seventh' dose is 4-24 mg, preferably 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg or 24 mg, preferably 4 mg, 18 mg or 24 mg.

25. The method according to any one of claims 23-24, wherein:
the method comprises administering to the subject in need thereof the first' dose of 1.5 mg of the compound of formula (I), or the pharmaceutically acceptable salt, amide or ester thereof, once weekly or once every two weeks;
after administering the first' dose for 2 weeks, administering the second' dose of 3 mg of the compound of formula (I), or the pharmaceutically acceptable salt, amide or ester thereof, once weekly or once every two weeks;
after administering the second' dose for 4 weeks, administering the third' dose of 5 mg of the compound of formula (I), or the pharmaceutically acceptable salt, amide or ester thereof, once weekly or once every two weeks;
after administering the third' dose for 4 weeks, administering the fourth' dose of 7 mg of the compound of formula (I), or the pharmaceutically acceptable salt, amide or ester thereof, once weekly or once every two weeks;
after administering the fourth' dose for 4 weeks, administering the fifth' dose of 9 mg of the compound of formula (I) or the pharmaceutically acceptable salt, amide or ester thereof once weekly or once every two weeks;
after administering the fifth' dose for 4 weeks, administering the sixth' dose of 11 mg of the compound of formula (I) or the pharmaceutically acceptable salt, amide or ester thereof once weekly or once every two weeks; and
after administering the sixth' dose for 4 weeks, administering the seventh' dose of 13 mg of the compound of formula (I) or the pharmaceutically acceptable salt, amide or ester thereof once weekly or once every two weeks.

26. The method according to any one of claims 1 to 25, **characterized in that**,
the compound of formula (I), or the pharmaceutically acceptable salt, amide or ester thereof, is administered parenterally, preferably subcutaneously.

27. The method according to any one of claims 1 to 26, **characterized in that**,
the compound of formula (I), or the pharmaceutically acceptable salt, amide or ester thereof, is administered for 12 months or more, preferably 16 months or more, preferably 18 months or more, preferably 24 months or more, preferably 28 months or more, preferably 29 months or more, preferably 30 months or more.

28. The method according to any one of claims 1 to 27, **characterized in that**,
the subject has a BMI of ≥18.5 kg/m², preferably has a BMI of 18.5-35 kg/m²; and/or
has an HbA1c of 7.0%-11%; and/or
has a fasting blood glucose of <15mmol/L.

29. The method according to any one of claims 1 to 28, wherein the compound of formula (I), or the pharmaceutically acceptable salt, amide or ester thereof, is administered in the form of a pharmaceutical composition comprising one or more pharmaceutically acceptable excipients.

30. The method according to claim 29, wherein the pharmaceutical composition comprises:
about 1.5 mg/ml, about 3 mg/ml, about 6 mg/ml, about 12 mg/ml, about 18 mg/ml, about 24 mg/ml, about 36 mg/ml, or about 48 mg/ml of the compound of formula (I), or the pharmaceutically acceptable salt, amide or ester thereof; preferably about 3 mg/ml, about 6 mg/ml, about 12 mg/ml, about 18 mg/ml, about 24 mg/ml, about 36 mg/ml, or about 48 mg/ml of the compound of formula (I), or the pharmaceutically acceptable salt, amide or ester thereof;
about 5 mg/ml, about 6 mg/ml, about 7 mg/ml, about 8 mg/ml, about 9 mg/ml or about 10 mg/ml of NaCl, preferably about 7 mg/ml of NaCl;
about 1 mM, 2 mM, about 3 mM, about 4 mM, about 5 mM, about 6 mM, about 7 mM, about 8 mM, 9 mM or about 10 mM of citric acid;
about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of a Na₂HPO₄ solution, preferably about 1.42 mg/ml of Na₂HPO₄; and
the pH of the pharmaceutical composition is about 7.2, about 7.3, about 7.4, about 7.5, about 7.6, about 7.7, about 7.8, about 7.9, about 8.0, about 8.1, or about 8.2, preferably about 7.3.
